# EUROPEAN PATENT SPECIFICATION

(11) **EP 3 452 929 B1**
(45) Date of publication and mention of the grant of the patent: **01.04.2020**
(21) Application number: 16723054.9
(22) Date of filing: 06.05.2016
(51) Int. Cl.: H04W 12/10, G16H 10/60

(54) **SYSTEM FOR EVALUATING TELEMETRY DATA**
SYSTEM ZUR BEURTEILUNG VON TELEMETRIEDATEN
SYSTÈME D'ÉVALUATION DE DONNÉES DE TÉLÉMÉTRIE

(43) Date of publication of application: 13.03.2019
(73) Proprietor: Innogy Innovation GmbH, 45139 Essen (DE)
(72) Inventor: STÖCKER, Carsten, 40724 Hilden (DE)
(74) Representative: Cohausz & Florack
(86) International application number: PCT/EP2016/060173
(87) International publication number: WO 2017/190795

(56) References cited:
- US-A1- 2006 010 090

## Description

The invention relates to a system for evaluating at least one telemetry data set relating to at least one first evaluation means. The invention also relates to a method for evaluating at least one telemetry data set, a peer-to-peer application for controlling an evaluation process of at least one telemetry data set and a peer-to-peer module.

The evaluation of telemetry data is generally known from prior art. Document US 2006/010090 A1 (BROCKWAY MARINA [US] ET AL) 12 January 2006 (2006-01-12) relates to an expert system for analysis or management of chronic medical conditions.

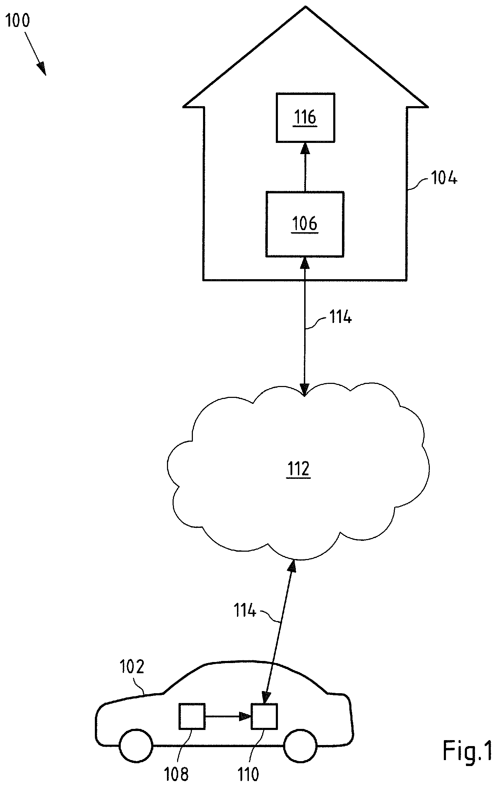

A typical prior art system is shown in figure 1. The depicted system 100 comprises a first entity 102 in form of a car 102. The car 102 comprises a sensor module 108 for detecting one or more telemetry parameters, such as a velocity or speed parameter, an acceleration parameter and/or a driven distance per time unit (e.g. km/a). The sensor module 108 can forward the detected telemetry data set to a communication module 110. The communication module 110 is configured to transmit the at least one telemetry data set e.g. via communication channels 114 and a standard network 112 to a server 106 of a second entity 104. The server 106 comprises evaluating means for evaluating the received telemetry data set. By way of example, evaluation can comprise a comparing step of a received parameter value with one or more predefined parameter threshold value(s).

The evaluation result can be forwarded to a further computing device 116 (e.g. a client) of the second entity 104. For instance, if the second entity 104 is an insurance entity 104, the insurance rate for the first entity 102 can be set in accordance with the evaluation result. By way of example, if the driven kilometers are below a specific threshold value, the insurance rate can be lower than the insurance rate if the driven kilometers are higher than said specific threshold value.

However, although a system 100 according to figure 1 is generally known in the art, such systems are little used in practice. A main problem of these systems is that a central system in form of one or more server(s) is used for evaluating telemetry data sets assigned to an entity, such as a user or an entity of a user. Thereby, many users have security concerns regarding the handling of their confidential telemetry data sets by one or more central server(s). In particular, with regard to the above example, one concern is that not only the evaluation result is used for determining the actual insurance rate but also the provided telemetry data set. In addition thereto, a further concern is that the provided telemetry data set is used for further applications by said second entity and/or a further second entity although the forwarding of said data is not allowed.

More particularly, common to all prior art systems for evaluating telemetry data are that a central subsystem, a central process and/or a central organization or instance is provided in order to manage and, in particular, evaluate the confidential telemetry data sets.

Technically speaking, a corresponding system for evaluating telemetry data is realized according to prior art by a client-server structure. The central organization or instance is created by one or a plurality of central servers. A server of this kind or a platform can be distributed and located on different computing devices, for example. This means that a virtual server can be realized by a cloud. For example, a centrally arranged database can be provided.

In particular, a central instance/server is configured to evaluate telemetry data relating to an entity. Thereby, the central server is used as a confidential instance for the telemetry data set relating to the entity. The central instance, such as a server or a platform, defines the rules, in particular, the evaluation rules. The central server ensures that the processes, such as an evaluation process, are carried out correctly for all entities involved. In other words, a central instance prevents tampering by one of the participating entities and/or by third parties.

The disadvantage of server-client structures of this kind, particularly the server (or platform), apart from the high transaction costs, is that the central instance or central server manages the confidential telemetry data of the users of the one more entities. A persistent problem affecting the central instance is that of protecting the confidential data stored on one server or a plurality of servers from access by unauthorized third parties. In particular, a high degree of security expenditure is required in order to prevent user data (e.g. registered peer-to-peer identifications), billing data, location data of the user, etc. from being tampered. This in turn leads to higher transaction costs. Another problem is to ensure that the raw data, i.e. the telemetry data, is not provided to any further entity but only the evaluation result.

Therefore, the object of the present invention is to provide a system for evaluating telemetry data which offers a higher level of security in a simple manner.

The object is solved according to a first aspect of the invention by a system for evaluating telemetry data according to claim 1. The system for evaluating telemetry data comprises at least one peer-to-peer module assigned to at least one first entity. The system comprises at least one peer-to-peer network with at least one peer-to-peer application. The peer-to-peer module is configured to transmit at least one telemetry data set relating to the first entity to the peer-to-peer application. The peer-to-peer application is at least configured to control evaluating means configured to evaluate the at least one telemetry data set according to at least one preset evaluation rule. The peer-to-peer application is configured to control the evaluating means such that at least one evaluation result is provided to at least one second entity without providing the received telemetry data set.

In contrast to the prior art, an evaluation process is securely controlled - without a central instance - by means of a peer-to-peer application of a peer-to-peer network. By the fact that instead of a central server or a platform, a peer-to-peer network (also called a framework) undertakes the, in particular, tamper-proof controlling of received telemetry data and of the evaluation process by means of a peer-to-peer application a high level of security is provided. In the case of a peer-to-peer network, high security standards are achieved in that all computers (peer nodes or simply nodes) in the peer-to-peer network, at least a part of the peer computers in the peer-to-peer network, monitor(s) at least the security of the evaluation process. Transaction costs can be reduced. No central, superior platform, server, cloud, etc. is required.

The system for securely evaluating telemetry data comprises at least one first entity and at least one second entity. The telemetry data to be evaluated is related to the first entity. This means that the data refers directly or indirectly to the first entity. An exemplified telemetry data set may comprise at least one telemetry parameter of the group comprising temperature of the entity, humidity of the entity, velocity of the entity, acceleration of the velocity, driven distance per time unit, distances to another vehicle at a particular velocity, geo-location of an entity, fuel charging levels, driver safety sensors, theft or collision prevention, all kind of data generated by an on-board or built-in unit of a vehicle, all kind of other environmental data gathered by a device, one or more personal or medical parameters (humans, animals) provided by at least one medical technology (medtech) device(s) and/or wearable(s) device and/or biological or geophysical sensor(s) and/or smart meter devices, etc.

An evaluation result of the evaluation process of at least one telemetry data set is at least provided to a second entity. The evaluation result is provided to the second entity without providing and disclosing, respectively, the received telemetry data set to the second entity. The first entity may be configured to decide if the raw data should be (partly, fully) disclosed or not.

It shall be understood that the evaluation result can also be provided to a further entity, such as the first entity. It shall be further understood that additionally at least one further telemetry data set related to a further entity, such as the second entity, can be evaluated.

Furthermore, the system comprises at least one peer-to-peer module. The peer-to-peer module is (uniquely) assigned to the first entity. This means that the peer-to-peer module can at least communicate and/or act on behalf of the first entity. Since the first entity can be assigned to one or more user(s), the peer-to-peer module can be (indirectly) assigned to the at least one user. On the other hand, the peer-to-peer module can be (directly) assigned to a user and via the user (indirectly) to a first entity of the user. A user may also be an entity.

For example, the peer-to-peer module can be integrated in the first entity or it can be formed by a separate processing device, such as mobile terminal, owned by a user of the first entity, or it can run on a remote processing device (e.g. a remote node in a data centre). In case of a remote processing device the first entity may have a secure communication channel to the processing device of the data centre or the communication is encrypted on an DAPP (decentral application) or peer-to-peer application level and the processing device itself may have a connection to the peer-to-peer network. In an alternative embodiment, the remote processing device may be a gateway to the peer-to-peer network. This means that the first entity has a secure access to the peer-to-peer network via a communication channel with the gateway.

It shall be understood that there may be further peer-to-peer modules. For instance, a further peer-to-peer module may be (uniquely) assigned to the at least one second entity and/or a further entity.

In comparison to a client-server network in which a server provides a service (i.e. conducting the evaluation process) and one or more client(s) use(s) the service, these roles are cancelled in the present peer-to-peer network. Each participant of the peer-to-peer network can use a service and the like and offer such a service. In particular, a peer-to-peer network is self-determined and/or self-organized (without any higher-level units). In the present case preferably each computer of the peer-to-peer network comprises a peer-to-peer application. In particular, the peer-to-peer module is configured to transmit telemetry data e.g. by sending one or more message(s) to the peer-to-peer application. The peer-to-peer module may be a peer of the peer-to-peer network.

The peer-to-peer application is configured to control an evaluation process conducted by an evaluating means. At least one evaluating means, controllable by the peer-to-peer application, may evaluate the at least one telemetry data set in accordance with at least one preset evaluation rule. The at least one evaluation rule comprises at least one evaluation instruction for generating an evaluation result. By way of example, the evaluation rule may be a comparison rule for comparing at least one telemetry parameter value of the at least one telemetry data set with at least one preset parameter threshold and/or with at least one telemetry parameter value of at least one further telemetry data set (e.g. of a further entity). The evaluation rule may be an algorithm executable by an evaluating means. Further evaluation means may comprise one or more evaluation rule for one or more of the following exemplified application(s):
- Telemetry data in form of vehicle usage data to calculate maintenance recommendations,
- Telemetry data in form of driver data to calculate an insurance premium,
- Telemetry data in form of IoT (internet of things) machine data to optimize usage of an machine or a system,
- Telemetry data in form of multiple machine data to optimize usage of a fleet,
- Telemetry data in form of wearable personal data to create health recommendations,
- Telemetry data in form of user or patient data comprising human genome data to conduct health diagnosis and/or
- Telemetry data to create a trigger or emergency message and/or and an actor.

An evaluation means may (additionally) include one or more compliance and/or auditing rule(s). This means that an evaluating result is (only) generated if one or more compliance and/or auditing rule(s) is/are fulfilled and/or an information about a check of one or more compliance and/or auditing rule(s) is/are generated with the evaluating result ("Compliance in Advance", "Auditing in Advance").

The peer-to-peer application is configured to control the evaluation process such that the raw data (i.e. the at least one telemetry data set) is not provided to any further entity. By means of the peer-to-peer application and at least a part of the nodes of the peer-to-peer network, it is ensured that only an evaluation result is provided to at least one second entity. Since the at least one peer-to-peer module assigned to the first entity is a peer of the peer-to-peer network it can monitor the use of the at least one provided telemetry data set. In other words, the peer-to-peer module assigned to the first entity is configured to monitor the provided telemetry data set relating to said first entity. Thus, an unauthorized transmission of said telemetry data set to any further entity can be prevented.

Preferably, the at least one telemetry data set, preferably each provided telemetry data set can be irretrievable deleted after the evaluation process. In particular, the at least one peer-to-peer module which provided at least one telemetry data set may be configured to monitor the deletion process. The correctness of the deletion process can be preferably monitored by at least a part of the nodes of the peer-to-peer network (without reading said deleted data set).

The list of all (anonymised or pseudonymised) participants or entities of the at least one peer-to-peer network can preferably be made known to each participant, so in particular to each entity of the present system, as a peer-to-peer identification, e.g. in the form of a communications address.

A peer-to-peer module and/or an evaluating means can be at least partially formed by a software module and/or at least partially formed by a hardware module. There may be two or more different peer-to-peer networks each configured for one or more different evaluation process(es).

As will be described in more details, telemetry data can be gathered from a variety of different entities and objects, respectively: vehicles, wearables, medtech devices, such as medtech devices for X-Ray, nuclear medicine, ultrasonic testing, cardiology analytics, nephrology analytics, tissue analytics, blood Analytics, breath / urine / stool analytics, DNA sequencing, DNA slicing, smart meter, IoT sensors of machines. Telemetry data can include measurement data, data about a telemetry device and/or other related (input) data such as biomarkers used in a medical analysis.

According to a first embodiment of a system of the present invention, the evaluating means may be further configured to generate at least one protocol message comprising protocol data about the conducted evaluation process. The peer-to-peer application (or a local algorithm controlled by the peer-to-peer application via an API) may be configured to provide the protocol message to at least one of the first entity and the second entity. In order to provide a proof, in particular, a cryptographic proof, for a correctly performed evaluation process, the evaluating means may be configured to generate at least one protocol message comprising protocol data about the conducted evaluation process. The protocol data comprises information which allows verifying the correctness of a conducted evaluation process by at least one of the first and second entities. With the provided proof a second entity can validate that the evaluation algorithm was executed correctly without disclosing the input data to the second entity. It may also be possible that correctness of a conducted evaluation can be verified by all nodes of a peer-to-peer application without having access to the input or raw data. For example, a privacy preserving protocol may be generated. Such a privacy preserving protocol method may ensure the privacy of the telemetry data and the correctness of code execution, in particular, of the one or more evaluation rules (SNARK verification is done via the smart contract on chain; cryptographic proof or verification data may be stored on chain or in a decentral file storage or decentral database or cloud storage). Preferably, the protocol message is provided to at least the sender of the telemetry data set (first entity) and/or to the recipient of the evaluation result (second entity). In addition, at least part of the nodes of the peer-to-peer network can verify the data of a protocol message, and thus, the correctness of the evaluation of the at least one provided telemetry data set.

Preferably, the at least one telemetry data set (or sub-set) and/or the output of an evaluating means e.g. including cryptographic proofs, preferably each or selected parts of each provided telemetry data set (or sub-set) can be stored in encrypted format to a data store (e.g. decentral file storage, BigchainDB or cloud). (Only) The first entity may have one or more cryptographic access key(s). By having the at least one key, the first entity may have full control about further usage of the encrypted telemetry data set(s) and/or the evaluating result(s) e.g. including the cryptographic proof. The first entity may e.g. decide if the encrypted data should be disclosed to one or more other entity(ies). This can be done by providing at least one encryption access key to the at least one further entity (selective transparency). Alternatively or additionally, the second entity may (only) have a cryptographic access key to disclose the evaluating result(s) to one or more other entity(ies) (selective transparency). This may be useful in case of any data needed to be audited by a third party.

According to a preferred embodiment of the system according to the invention, at least one peer-to-peer module assigned to at least one of the first entity and the second entity may be configured to cause generating of at least one evaluation transaction agreement about at least the terms for input of the telemetry data set and/or the at least one evaluation rule and/or the terms of output of the evaluation result. The evaluation transaction agreement may further include one or more term(s) e.g. about the storage location of a telemetry data set and/or evaluation result and/or protocol data e.g. including (cryptographic) proofs, which encryption rule(s) is/are applied and/or with which entity(ies) encryption key(s) can be exchanged.

Preferably, a peer-to-peer module, such as a peer-to-peer module assigned to the first or second entity, may initiate or cause the generation of an evaluation transaction agreement. In other words, the conduction of an evaluation of telemetry data may require that a peer-to-peer module is configured to cause or trigger a previous generation of an agreement between e.g. the at least one provider of the telemetry data set(s) and the at least one recipient the evaluation result. The evaluation transaction agreement can comprise data, such as involved entities (e.g. identifications of the first and/or second entity(ies)), at least one evaluation rule and/or data about the input of telemetry data (e.g. kind of input or kind of telemetry data) and/or the output of an evaluation result (e.g. kind of output or kind of evaluation result). Preferably, based on these data an evaluating means can be configured and created, respectively. Without a central instance two entities can cause the generation of an evaluation transaction agreement in a simple manner. Thereby, the entities can set the evaluation rule(s) on their own.

Generally, a first entity may be any entity, such as a building, mobile unit (e.g. vehicle or mobile terminal), a medtech device, a wearable, a smart meter, an IoT sensor, an embedded device, other units, user, etc., as long as the entity has at least one telemetry parameter to be evaluated. An entity may also be formed by two or more of the previous examples. Preferably, a first entity can be assigned (e.g. comprise or be connected with) to a sensor module configured to detect or measure at least one telemetry parameter. Detecting a telemetry parameter means in particular that a current value of said telemetry parameter is detected.

In a preferred embodiment, the at least one first entity may be a vehicle comprising at least one sensor module configured to detect at least one telemetry parameter relating to the vehicle. The detected telemetry parameter may be provided to the peer-to-peer module assigned to the vehicle. Based on the at least one provided telemetry parameter the peer-to-peer module may generate a telemetry data set, which can be transmitted to the peer-to-peer application. Exemplified vehicles are cars, trucks, ships, railway vehicles, planes, bicycles, motor bicycles, drones, mobile machines, boats, submarines, space vehicles, etc. Preferably, the vehicle can comprise the at least one peer-to-peer module assigned to said vehicle. In one particular embodiment, the peer-to-peer module may be attached to the diagnosis connector (e.g. On-Board Diagnosis (OBD) connector in case of cars). The peer-to-peer module may be additionally configured to collect telemetry parameter from one or more sensors of the sensor module of the vehicle. For instance, based on a generated evaluation transaction agreement, the peer-to-peer module may collect the respective telemetry data and may send a telemetry data set comprising said parameters to the peer-to-peer application.

Furthermore, according to an embodiment of the present invention, the peer-to-peer application may comprise the evaluating means. Such calculating means can be securely controlled. Alternatively or additionally, at least one database controllable by the peer-to-peer application may comprise the evaluating means. Preferably, the database may be formed as a decentral file system (such as IPFS) or decentral distributed database (such as BigchainDB) controlled by the peer-to-peer application, such as by a smart contract of the peer-to-peer application. The smart contract may be generated as a result of a generated evaluation transaction agreement. By providing an off chain processing of the telemetry data sets, a high and particular powerful processing power for conducting one or more evaluation processes and/or computing cryptographic proof(s) can be provided.

Further, at least one computer node of the peer-to-peer network may comprise the evaluating means. Preferably, one or more particular powerful nodes may comprise one or more (different) evaluating means. For instance, besides the peer-to-peer application at least one evaluating means arranged within a secured environment (or trusted execution environment or measured launch environment) of the node can be provided (an example for secured environment: Intel SGX technology). For instance, as a secured environment a node (or another unit, such as a IPFS or any other peer-to-peer network node which includes a secured environment or is at least has a (remote) connection to a secured environment) can have a secure hardware enclave for attestation and sealing that cannot be manipulated by other software code running on the devices. Within this secured environment the at least one evaluation process can be conducted.

It shall be understood that the evaluating means may be formed by two or more separate modules, wherein the two modules may be comprised by two different units, such as the peer-to-peer application and a database.

According to a preferred embodiment of the present system, the evaluating means may be formed by a private smart contract comprising the at least one evaluation rule. A private smart contract e.g. verified and / or controlled by a smart contract, is particular suitable for conducting a secure evaluation of one or more telemetry data set(s). A private smart contract may only be inspectable by authorized peers (for instance, in accordance with an evaluation transaction agreement).

A smart contract (e.g. running on a peer-to-peer application, in particular, chain) and a 'private contract' (running e.g. off chain or on a subset of nodes) may be generated as a result of a generated evaluation transaction agreement. The generation of an evaluation transaction agreement may be performed by a compiling means. A compiling means may be configured to create a smart contract and preferably a private contract code. The created code can be deployed on chain (smart contract) and preferably on a device acting as a 'minimally trusted manager' (running a private contract) and/or using an interactive protocol deployed among two or more entities to make use of secure multi-party computing (sMPC).

The minimally trusted manager may ensure efficient execution of code and/or protocol(s). Such a manager can read the transaction, but not manipulate the outcome. A peer-to-peer application may have access to several minimally trusted manager entities and may e.g. use several minimally trusted managers to avoid or at least reduce risk(s) of a corrupt manager.

Code running on a 'minimally trusted manager' entity may use trusted computing hardware (e.g. Intel SGX) for device based 'Attestation' and 'Sealing'. In such a set-up a trusted computing base (TCB) may protect one or more secret(s) and/or the software and/or data inside an enclave and container, respectively, by hardware enforced access control policy(ies). With this set-up software data can be remotely deployed to an enclave and it is ensured that secret(s) is/are protected and/or securely stored in a (non-volatile) memory. In this process the one or more of the following steps may be conducted by the peer-to-peer application for deploying off-chain code and/or data: Enclave Launch, Attestation, Provisioning, Sealing/Unsealing and Software Upgrades and / or Data Transfers. A smart contract may control the process to deploy (private contract) code and data to such an enclave.

When ownership of this trusted computing entity or enclave is transferred by an entity owner, secret(s) available during the ownership period may be made inaccessible or may be erased by an (proper) algorithm. An enclave itself may be registered as a smart asset in the peer-to-peer application and/or it may (also) have a smart contract linked to the peer-to-peer application. Access and/or ownership of such an enclave can be shared and/or transferred via the peer-to-peer application. This means in particular that available resource(s) of enclave(s) (e.g. running in a datacentre and/or on a high performance node of the peer-to-peer network and/or on a single home device and/or a marketplace of enclaves) can be easily and securely made available to a smart contract. This smart contract may run an "evaluation transaction agreement" by using a secure environment /enclave for private contract code execution. These types of smart contracts can dynamically allocate enclaves on demand for private contract execution. An enclave registry, marketplace, performance/SLA monitoring and/or reputation system can be established in order to enable evaluation transaction agreement(s) or smart contract(s) to select, buy and allocate required private contract execution capacity. When smart contract and/or private contract code is generated for evaluation transaction agreement(s) private contract (or off-chain or enclave) execution capacity can be calculated. During the deployment process off-chain or enclave execution capacity can be bought and/or allocated using information from above mentioned enclave registry, performance/SLA and reputation system.

The calculation of private contract(s) and/or protocol(s) comprising cryptographic proof(s) may make use of specific hardware and/or software configurations that are preferably optimised for executing private contract(s) and/or setting up cryptographic proof system(s) and/or computing cryptographic proof(s) (e.g. high performance private contract and/or cryptographic proof system computation module(s) running in a data centre that is connected via a API to one or more nodes of a peer-to-peer network).

In case of sMPC, privacy enforcing computation may be conducted on a (sub-)set of nodes of the peer-to-peer network by using a sMPC protocol (such as Enigma). The computation step(s) may be public verifiable. The sMPC nodes may be run on specific hardware to support high performance execution of the (privacy enforcing) computation step(s). Telemetry data may not be disclosed and correct execution may be validated by one or more entity(ies) of a peer-to-peer network.

It shall be understood that a high performance private contract or sMPC node(s) can be used for use cases when privacy is not required in order to execute algorithms off-chain and to control proper execution on-chain by a peer-to-peer application. This can be particularly useful when (resource intensive) optimisation algorithms are executed off-chain (for performance or scalability reason(s)) and their execution needs to be done in a trustless way.

In order to associate an evaluation result of a telemetry data set to the provider of said telemetry data set, according to a further embodiment, the at least one telemetry data set relating to the first entity may be associated with a unique peer-to-peer identification of the first entity. The at least one evaluation result may be associated with the unique peer-to-peer identification of the entity. The peer-to-peer identification may be a unique peer-to-peer identification within the peer-to-peer network. Preferably, each participant can comprise such an unique peer-to-peer identification, like an unique code or the like. In order to identify the sender of a telemetry data set, preferably each telemetry data set may be associated with the peer-to-peer identification of the first entity to which the telemetry data set is related to. This allows that also an evaluation result can be associated with the sender of the telemetry data set.

Further, in order to provide preferably each of the participants of the peer-to-peer network with a unique peer-to-peer identification, according to a further embodiment, an entity (e.g. vehicle, mobile terminal, user, wearable, medtech device, IoT sensor, embedded device, etc.) can be registered in the peer-to-peer application as e.g. a so called smart asset. During this process a smart contract for an entity can be set-up to store value and/or provide access and/or transfer ownership. According to an embodiment of the system according to the present invention, a peer-to-peer module assigned to an entity may be configured to cause a registration of the entity (or peer-to-peer module) in the peer-to-peer application (or network) by transmitting a registering message comprising at least an identification assigned to the entity. The identification might be already a peer-to-peer identification or another identification suitable to uniquely identify the entity, such as a serial number or a smart asset hash of e.g. the entity, the user's name of the entity, a communication address of a communications device of the entity, etc. If e.g. the identification is not already a peer-to-peer identification, e.g. if the identification is a name of the user, the peer-to-peer application may be configured to generate a unique peer-to-peer identification for the respective entity (according to preset rule(s)). In particular, the peer-to-peer application may register the respective entities by storing at least the peer-to-peer identification in the peer-to-peer application or a database controlled by the peer-to-peer application.

Prior to the registration of an entity at least part of the peers of the peer-to-peer network may check whether the registering requirements (such as entity specifications or stored minimum value or valid signatures or compliance requirements) predefined by the peer-to-peer network are met by the entity requesting registration. In order to perform the check, preferably, further data may be included in the registering message. In particular, the peers of the peer-to-peer network may provide registering rules or registering requirements which must be fulfilled by an entity to be regarded as a trustful entity. By way of example, each entity to be registered must comprise an account and/or a particular amount of a cryptocurrency. Other rules/requirements may be individually defined by the peers of a peer-to-peer network. E.g. it may be necessary that a new entity must be recommended by an entity which is already a participant of the peer-to-peer network. In addition, it may be necessary that this participant must have a reputation factor which increases a predefined minimum reputation factor.

It shall be understood that an evaluation means may need (external) data. These data can be provided from one or more smart oracle(s). A smart oracle may be registered - after validation of registering requirements - as a participant of the peer-to-peer application.

Furthermore, it may be advantageous if in the registering process also further e.g. technical details of the entity e.g. about the kind of telemetry parameters related to the entity, the kind of entity, etc., can be stored together with the respective peer-to-peer identification in the peer-to-peer application or the database accessible by the peer-to-peer application.

According to a preferred embodiment, the peer-to-peer module assigned to the first entity may comprise encrypting means configured to encrypt the at least one telemetry data set. By encrypting the telemetry data set prior to transmitting the at least one telemetry data set it can be ensured that the content of the telemetry data set, i.e. the detected values, cannot be read or otherwise used by an unauthorized third unit or person. Further, the peer-to-peer module may be preferably configured to transmit the encrypted telemetry data set together with the identification to the peer-to-peer application.

In addition, according to a further embodiment of the present invention, the peer-to-peer application may be configured to forward the received encrypted telemetry data set to the evaluating means based on the peer-to-peer identification associated with the received telemetry data set. For instance, if the peer-to-peer application controls two or more evaluating means, the peer-to-peer application can use the peer-to-peer application to select the evaluating means responsible for the received telemetry data set. By way of example, a previously generated evaluation transaction agreement may comprise respective information such that the peer-to-peer application is capable of forwarding the telemetry data set to the agreed evaluating means.

Furthermore, a peer-to-peer application might be capable of dynamically allocating evaluation means in order to optimise resource usage of evaluating means. It might also be capable to check if evaluating means fulfil required performance and/or service levels, if they are over- or underutilised or if there are malfunctions or degradations. If this is the case the peer-to-peer application might be capable to reallocate or redistribute evaluating means (load balancing of evaluating means).

Preferably, the evaluating means may comprise at least one decryption means for decrypting the forwarded telemetry data set and/or smart oracle input data. Only the evaluating means (e.g. besides the sending entity) may comprise a decryption means having one or more suitable decryption key to decrypt the received telemetry data set. Preferably, a provided telemetry data set is only decrypted within a previously described secured environment. This improves further the security of the present system.

What is more, according to another preferred embodiment, according to a preferred embodiment, the peer-to-peer application may be configured to perform at least one predefined action depending on the evaluation result. The conduction of the predefined action can be automatically conducted after the generation of a respective (encrypted) evaluation result and/or triggered by at least one peer-to-peer module, such as the peer-to-peer module assigned to the second entity. For instance, the at least one predefined action can be defined within an evaluation transaction agreement. In particular, in an evaluation transaction agreement, it can be defined that one or more specific evaluation result(s) cause the (automatic) conduction of one or more defined action(s). Exemplified actions are financial transactions, trigger or sending emergency message(s) and/or user request message(s) (e.g. for disclosing telemetry data while receiving a financial payment for the data) and/or control actions for controlling one or more actor(s) e.g. of the respective first entity. An actor may be configured to influence at least one telemetry parameter previously evaluated by the evaluating means.

According to a further preferred embodiment, the at least one first entity is a medical technology device comprising at least one sensor module configured to measure at least one telemetry parameter relating to at least one user of the medical technology device. The telemetry data may be indirectly related to the first entity in form of the medtech device. The medtech device may be a wearable device or a stationary device. The sensor module may comprise one or more sensors for measuring and analysing a probe and/or sample and/or body data. For instance, body data, such as heart rate, blood pressure, movement data, can be measured and analysed by a sensor module of said entity. Alternatively or additionally, the entity may be configured to provide X-Ray data, nuclear medicine data, ultrasonic testing data, cardiology analytics, nephrology analytics, tissue analytics, blood analytics, breath analytics, DNA Sequencing, DNA Slicing and/or Biomarkers used.

Preferably, the measured telemetry parameter may be provided to the peer-to-peer module assigned to the medical technology device. The peer-to-peer module assigned to the medical technology device may transmit said at least one telemetry parameter to the peer-to-peer application for evaluating the data, as described before.

In addition, the measured data can be encrypted by the peer-to-peer module assigned to the medical technology device and/or the user by using encrypting key(s) controlled and/or owned by the patient. The encrypted data can be stored in a secured environment, such as an IPFS, BigchainDB, cloud storage and/or in a smart contract (as described hereinbefore). Such a storage unit can be used for generating a secure and (only) user or patient controlled database (by means of the peer-to-peer application and network).

The user can provide access, in particular by means of a peer-to-peer module assigned to the user, to at least part of the data to further entity(ies), such as medical practitioner. The user can provide also, in particular by means of a peer-to-peer module assigned to the user, write permission of the database to further entity(ies), such as medical practitioner. For instance, one or more encryption key(s) can be disclosed to the at least one further entity. On the one hand, patient data is securely stored and (fully) controlled by the patient itself. On the other hand, all relevant patient data is available, if needed and allowed by the patient.

According to one embodiment, the telemetry parameter may be at least part of the DNA of the user of the medtech device. Such a telemetry parameter may be analysed by means of a smart contract and/or a private contract preferably comprising algorithm(s) and/or artificial intelligence. The DNA (portion) can be analysed in a secure way and under control of the peer-to-peer application. Only the analysis or evaluation result can be provided to a second entity without providing the DNA (portion) itself.

According to a further embodiment, the system may be a treatment life cycle monitoring system. In particular, for detecting side effect(s) of a (prescript) drug and/or healing effects, the above described system can be implemented as follows:
By means of one or more medtech device(s), telemetry data, such as patient master data, anamneses, analysis data, diagnosis, diagnostic findings and/or prescriptions can be provided to the peer-to-peer application. Then, also by means of one or more medtech device(s) (including wearable(s) and other sensors), telemetry data, such as the actually taking of a specific dose at a particular time, the behaviour data of the patient (doing sport, resting, smoking, drinking alcoholics, etc.) can be provided to the peer-to-peer application by means of a peer-to-peer module, as previously described.

At least some of the telemetry data may be manually input into a computer or the like and forwarded by a peer-to-peer module of said computer. The telemetry data may be provided, e.g. input by the patient itself or a third (authorized) person, such as a medical practitioner.

The provided telemetry data can be evaluated in order to determine healing and/or side effects of at least one drug. Only the evaluation result (e.g. without an identification of the patient and/or the actually provided telemetry data) may be forwarded to a second entity, such as the provider of the drug. The above described system and medtech device is independently of the former description an invention.

According to a further embodiment, the at least one peer-to-peer application can be a decentralized register or a shared database storing transaction agreement(s) and data with given certain proofs or signatures. In addition to e.g. evaluation transaction agreement(s) and data, the decentral register can store computer code acting as e.g. means for controlling evaluating means or sending/receiving data. In particular, the code can be invoked by a transaction to the address of the code in so called 'smart contracts'. This code is processed on the nodes of decentral register.

A decentralized register can be readable at least by a part of the participants of the peer-to-peer network. In particular, every computer node including e.g. the at least one peer-to-peer module assigned to an entity can comprise the peer-to-peer application. The decentralized register, at least the public part (i.e. maybe without private contracts) may be read at least by each participant of the peer-to-peer network. In particular, all peer-to-peer modules and all other computers of the peer-to-peer network can preferably read all information in the peer-to-peer application formed as a register. Preference is also that all peer-to-peer modules and all other computers of the peer-to-peer network can send messages to or write messages to the peer-to-peer application. A message or transaction sent to a smart contract may start the execution of a code of the smart contract while using data stored in the smart contract.

The peer-to-peer application can be built upon the following elements: peer-to-peer network, Consensus System / Protocol, Data Structure, Merkle Trees, Public Key Signatures, Byzantine Fault Tolerance. It may replicate data based on a consensus principle. It may be auditable and traceable.

In a simple way information can be made available to preferably all participants. This may allow to carry out a review of the information stored in the decentral register or the code executed in the decentral register. Particularly preferably, each computer in the peer-to-peer network can be configured to review new information, in particular, based on older information stored in the peer-to-peer application.

Moreover, preferably each computer can in each case comprise the complete data content, but include at least a portion of the data contents of the peer-to-peer application, in particular of the decentral register. For example, it may be provided that after a positive verification of written information in the peer-to-peer application this information is saved by all computers, at least by a part of the computers. The tamper resistance of the data stored in the peer-to-peer application can thereby be further improved. An evaluating process can be securely controlled.

In order to store new information in a tamper-proof way, the peer-to-peer application can comprise encryption means and/or signature means and/or verification means, wherein at least one of the encryption means and/or signature means and/or verification means is configured to store data, such as evaluation transaction(s) or evaluation result(s). In particular, it can be provided that by the hash function a link is established with at least one previously stored information in the decentral register. Further data, such as request messages, ordinary, contextual and/or transaction data of an entity, such as a vehicle, can be stored.

The peer-to-peer application may be formed by a Directed Acyclic Graph (DAG). A directed acyclic graph, such as IOTA or Tangle, means that blocks (or nodes of the graph) are coupled to each other via directed edges. Thereby, direct means that the (all) edges have (always) a same direction similar to time. In other words, it is not possible to step back. Eventually, acyclic means that loops do not exist.

In a particularly preferred embodiment of the present system, the peer-to-peer application can be a block chain or decentral ledger comprising at least two blocks coupled to each other (e.g. Ethereum Block chain with Smart Contracts). The block chain technology or "decentral ledger technology" is already used in the payment by means of a crypto currency, such as Bitcoin. It has been recognized that by a particular configuration of a block chain, at least the correctness of a received or provided data and of conducted processes, such as an evaluation process, can be checked without the need of a central server. In addition, the block chain can be used to generate predefined action(s) caused by at least one peer-to-peer module and/or an evaluation result in a tamper-proof manner. The block chain according to the present embodiment is particularly a decentralized, peer-to-peer-based register in which all data related to at least one evaluation process can be logged. A block chain is particularly suitable as a technical means to replace a central entity/server in a simple and secure manner.

In alternative embodiments of the peer-to-peer application, the block chain can be a permissionless or permissioned block chain. In a specific case the block chain can be public, consortium or private block chain.

In a further embodiment, the peer-to-peer application can be formed by multiple block chains which are connected via mechanisms such as side chains or smart contracts.

Data of the peer-to-peer application can be stored on the "decentral ledger technology" and/or the decentral ledger steers (encrypted) data storage accessible via the internet and preferably in de-central data storage and database, respectively, such as Interplanetary File System (IPFS) or in a distributed Blockchain database (e.g. BigChainDB). Access to the encrypted data to third party entities is managed via identity and access management transactions or smart contracts via the block chain.

In addition, data feeds can be provided by the peer-to-peer application (so called "smart oracles"). Data can be captured from trusted sources off-chain and stored on the block chain or stored via the block chain on a decentral data storage entity.

Information among peer-nodes can be exchanged by a peer-to-peer messaging system. This means a peer node can send a message to another peer node to submit an information or to trigger an action. Messages can be clear text, signed and/or encrypted. This means that not all data exchanged among peer nodes must be stored on the block chain.

In a further embodiment, the at least one peer-to-peer network can be formed by a plurality of computer nodes and a peer-to-peer module, such as the peer-to-peer module of an entity, a peer-to-peer module of a mobile terminal, etc., is only configured to communicate with the plurality of computer nodes. In other words, the peer-to-peer module is not a computer node of the peer-to-peer network but only a participant. Such a peer-to-peer module does not comprise the peer-to-peer application but only provides an interface module, such as an application programming interface (API), and a decentral application for communication with the computer nodes of the peer-to-peer network or the peer-to-peer application, such as a block chain or a smart contract on the block chain. For instance, such a peer-to-peer module can either send clear text or encrypted information or generate a secure connection (e.g. tunnel) to a peer-to-peer gateway (or so called "remote node") in order to communicate with the peer-to-peer network. This allows reducing the required processing power of the peer-to-peer module.

In one implementation of the peer-to-peer network, there can be only one validating peer or full node, e.g. only one node can be configured to perform a validation process, and one or more observing nodes. An observing node can validate transactions to establish a trust level but does not validate all transactions which is done by the validating peer.

In an alternative embodiment, the peer-to-peer module is one of the computer nodes. In this case, the peer-to-peer module comprises at least a part of the peer-to-peer application. In particular, the peer-to-peer module can comprise preferably the total data content of the peer-to-peer application or can access the information stored in another node. For instance, the peer-to-peer module might be a so called "light node" or a decentral application (DAPP) connected to a remote node.

It is noted that in the present case, according to an embodiment, the peer-to-peer module comprises at least an API configured to communicate with the peer-to-peer application, such as the block chain. In addition to the API, the peer-to-peer module comprises a decentral application of software comprising local algorithms at least configured to create and transmit data, transactions or the measured quantity parameter to the peer-to-peer application via the API. The decentral application so called "Dapp" is at least configured to process and transmit the meter data.

Preferably, the data is signed or encrypted or can be transmitted via a cryptographically secured tunnel or a secured internet connection to a peer-to-peer node running the peer-to-peer application, such as the block chain. In another particular embodiment, also the peer-to-peer application itself is implemented in the peer-to-peer module, i.e. the peer-to-peer module is a node of the peer-to-peer network comprising the decentral application, the API and the peer-to-peer application, such as the block chain or decentral ledger.

Further processing of data, such as evaluating at least one motion parameter, can be done on-chain or off-chain. Off-chain data processing, evaluating and/or validation can be managed by the peer-to-peer application, like the code on the block chain. Powerful means in particular a high computing power. In other words, in the present case a valid entry in the peer-to-peer application, such as a block chain, is assumed if (only) a part of the peers comes to a positive result. It shall be understood that only a single, especially particularly powerful peer can perform the processing or validation process. In an alternative embodiment, the block chain may delegate computing tasks to one or more resources. It either trusts one resource or it applies a consensus principle while delegating similar processing tasks to many resources.

Data and transactions stored on the block chain do not provide "transactional privacy". Transactions between pseudonyms may be (often) stored in clear text on the block chain. In some cases data stored on the block chain are encrypted and the keys may be handled via the block chain. Transactions between pseudonyms are stored in clear text on the block chain. Privacy preserving, secure transactions or execution of computer code can be achieved with cryptographic tools such as zero knowledge (zk) proofs or zk Succinct Non-interactive Arguments (zk-SNARK). Transactions or algorithms are separated into two parts: a smart contract on the block chain and a private contract. A privacy preserving protocol ensures the privacy of data and the correctness of code execution (SNARK verification is done via the smart contract on chain). The private contract computation can be done by a set of nodes, off-chain computers or done in measured launch environment or a secure HW enclave for attestation and sealing that cannot be manipulated by other software code running on the devices. In an alternative embodiment secure Multi-Party-Computing (sMPC) systems can be used for transactional privacy. Examples for privacy preserving protocols and computation are HAWK and MIT Enigma.

With zero knowledge proof (zk Proofs) the parties can see that the algorithm is executed correctly in a private contract, but the input data are not disclosed to the party. In addition selective privacy can be achieved by sharing keys to decrypt transactions for reporting and auditing purposes.

Similarly, in an alternative (not shown) embodiment a particularly large peer-to-peer network may be divided in two or more (physical or logical or dynamically virtual) clusters. In a corresponding peer-to-peer network, for example, a validation may only be carried out by the members of one cluster (a subset of nodes; e.g. sharding of a block chain to improve the scalability)). In a further embodiment, the peer-to-peer application can be formed using multiple block chains. These block chains are connected via frameworks such as sidechains.

Financial values can be (instantaneously) exchanged with a transaction via a cryptocurrency. In an alternative embodiment micropayment channels are used for a constant payment stream that can be handled party off-chain to reduce the amount of on-chain transactions. In a further embodiment, so called state channel(s) or state network(s) (e.g. Raiden Network) may be used to exchange digital tokens off-chain in a secure way. Opening and/or closing of a state channel may be registered on the block chain. This means that individual transactions may not be stored on the block chain in order to improve scalability and avoid movement tracking of pseudonyms on the block chain. According to the present invention, a man-in-the-middle is not necessary. Fully automated processes from authentication to charging and billing can be provided.

In a further embodiment, at least one entity of the system for evaluating telemetry and diagnosis data may have a digital wallet to exchange value in form of digital tokens and/or cryptocurrency.

In a further embodiment, at least one financial and/or evaluating transaction among at least two entities and/or users may be secured via at least one smart contract. A transaction may invoke the execution of the smart contract and/or a private contract. With this transaction a deposit or escrow in form of a cryptocurrency may be stored in the smart and/or private contract by the first and/or second entity. Further handling of the deposit might depend on terms specified in the smart and/or private contract. At the end of specified terms of an evaluating transaction agreement all remaining funds may be sent back to the entity that provided the deposit. Overall the logic described above may provide a secure payment process with no middle man involved and build-in transactional privacy for exchange of financial values.

The peer-to-peer application can be connected to a central system (e.g. central system connected to a peer-to-peer module or a remote node). Central systems can provide data or parameters that will be used by smart and/or private contracts (e.g. authority reporting, compliance parameters, legal requirements, technical system parameters, contract database of an insurance company, pharmaceutical system or medical system).

A further aspect of the present invention is a method for evaluating at least one telemetry data set. The method comprises
- transmitting at least one telemetry data set relating to at least one first entity from a peer-to-peer module assigned to the first entity to at least one peer-to-peer application of at least one peer-to-peer network,
- at least controlling an evaluation process of the telemetry data set by the peer-to-peer application, and
- providing at least one evaluation result by the peer-to-peer application to at least one second entity without providing the received telemetry data set.

The method can in particular be carried out by using a previously described system.

Another aspect of the present invention is a peer-to-peer application. The peer-to-peer application comprises means for receiving at least one telemetry data set relating to at least one first entity. The peer-to-peer application comprises means for controlling evaluating means configured to evaluate the at least one telemetry data set according to at least one preset evaluation rule. The peer-to-peer application comprises means for providing at least one evaluation result to at least one second entity without providing the received telemetry data set.

A still further aspect of the present invention is a peer-to-peer module assignable to at least one first entity. The peer-to-peer module comprises at least one input means configured to receive at least one telemetry data set relating to the first entity. The peer-to-peer module may comprise at least one encrypting means configured to encrypt the telemetry data set. The peer-to-peer module may comprise at least one communicating means configured to transmit the encrypted telemetry data set together with a unique peer-to-peer identification assigned to the first entity to at least one peer-to-peer application of at least one peer-to-peer network.

A still further aspect is a medical technology device comprising at least one previously described peer-to-peer module.

The features of the methods, systems, modules, peer-to-peer applications and computer programs can be freely combined with one another. In particular, features of the description and/or the dependent claims, even when the features of the dependent claims are completely or partially avoided, may be independently inventive in isolation or freely combinable with one another.

These and other aspects of the present patent application become apparent from and will be elucidated with reference to the following figures. The features of the present application and of its exemplary embodiments as presented above are understood to be disclosed also in all possible combinations with each other.

In the figures show:
- Fig. 1: a schematic view of an embodiment of a system according to prior art,
- Fig. 2: a schematic view of an embodiment of a system for securely evaluating telemetry data according to the present invention,
- Fig. 3: a schematic view of a further embodiment of a system for securely evaluating telemetry data according to the present invention,
- Fig. 4: a schematic view of a further embodiment of a system for securely evaluating telemetry data according to the present invention,
- Fig. 5: a schematic view of an embodiment of a peer-to-peer application according to the present invention,
- Fig. 6: a schematic view of a further embodiment of a system for securely evaluating telemetry data according to the present invention,
- Fig. 7: a diagram of an embodiment of a method according to the present invention, and
- Fig. 8: a schematic view of a further embodiment of a system for securely evaluating telemetry data according to the present invention.

Like reference numerals in different figures indicate like elements.

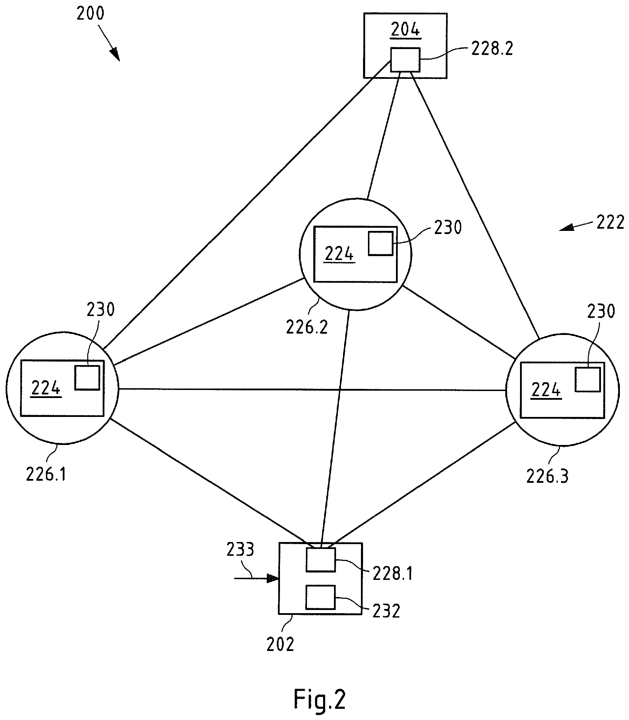

Figure 2 shows a first embodiment of a system 200 for evaluating telemetry data according to the present invention. The depicted system 200 comprises two entities 202, 204, a first entity 202 and a second entity 204. It shall be understood that other variants of a system according to the invention may comprise two or more first entities and/or two or more second entities and/or other entities.

At least one telemetry parameter related to the first entity 202 may be provided. By way of example, a sensor module 232 may be arranged in the first entity 202. The sensor module 232 may comprise one or more sensors configured to detect or measure one or more telemetry parameter(s) (e.g. a temperature related to the entity).

In addition (or alternatively), at least one telemetry parameter related to the first entity 202 may be provided from an external source 233. For instance, a temperature (outdoor temperature) can be provided by an external provider (e.g. meteorological service provider).

A substantial difference compared with prior art systems, such as system 100 according to figure 1, is that no central instance (and/or third party organization) is provided. In the present case, the system 200 comprises a peer-to-peer network 222 or a computer-computer network 222. The peer-to-peer network 222 comprises a plurality of nodes 226.1, 226.2, 226.3 or computers 226.1, 226.2, 226.3. A peer-to-peer network 222 is characterized in the present case in that each node 226.1, 226.2, 226.3 and/or participant 228.1, 228.2 is preferably connectable at least to every other node 226.1, 226.2, 226.3 and/or participant 228.1, 228.2. For instance, at least one physical standard network (wired and/or wireless) can be used for connection. For communicating via the at least one physical standard network suitable transceiver modules may be arranged in the respective entities.

In addition, the computers 226.1, 226.2, 226.3 have equal rights, something which distinguishes them from a server-client structure.

The depicted nodes 226.1, 226.2, 226.3 (each) comprise a peer-to-peer application 224. As can be seen from figure 2, the same peer-to-peer application 224 is preferably implemented on each node 226.1, 226.2, 226.3. The peer-to-peer application 224 may preferably be a public register 224 that can, in particular, be inspected by all participants 226.1, 226.2, 226.3, 228.1, 228.2 (not only the nodes 226.1, 226.2, 226.3) of the peer-to-peer network 222. Each node 226.1, 226.2, 226.3 preferably has the (entire) public register 224. It may also be envisaged that only part of the register can be provided on a node (light node). In a particularly preferred embodiment, the peer-to-peer application 224 may be a block chain 224 which will be explained in more details hereinafter.

Further, the peer-to-peer application 224 is configured to control evaluating means 230. In the present embodiment, the peer-to-peer application 224 comprises the evaluating means 230. Although a peer-to-peer application 224 may be generally a public register 224, it may comprise private means 230 which are not inspectable by the participants 226.1, 226.2, 226.3, 228.1, 228.2.

The evaluating means 230 may be preferably formed by a private smart contract 230 configured to evaluate received telemetry data set(s) according to at least one preset evaluation rule.

In addition, it can be seen from figure 2 that in the present embodiment the at least one first entity 202 comprises a peer-to-peer module 228.1. Preferably, the peer-to-peer module 228.1 and/or a sensor module 232 assigned to the entity 202 can be fixedly integrated in the entity 202. In other variants, at least parts of the modules 230, 228.1 can be formed as separate units.

It shall be understood that an evaluating means 230 can be part of the first or second entity 202 or 204. It shall be further understood that an evaluating mean 230 can be connected to a smart oracle in order to get access to further (external) data.

A peer-to-peer module 228.1 is (generally) configured to communicate at least with the peer-to-peer network 222, i.e. the nodes 226.1 to 226.3 of the peer-to-peer network 222. In other words, the peer-to-peer module 228.1 or the entity 202 corresponding or assigned to the respective peer-to-peer module 228.1 is at least a participant of the peer-to-peer network 222. Preferably, all participants 226.1, 226.2, 226.3, 228.1, 228.2 (including all nodes) of the peer-to-peer network 222 are known to each participant 226.1, 226.2, 226.3, 228.1, 228.2 of the peer-to-peer network 222.

In the present case, the at least one peer-to-peer module 228.1 is not a node of the peer-to-peer network 222 but only a participant 228.1 (same applies for the further peer-to-peer module 228.2 of the second entity 204). While the nodes 226.1, 226.2, 226.3 or computers 226.1, 226.2, 226.3 in the peer-to-peer network 222 comprise at least a part of the peer-to-peer application 224 a participant of a peer-to-peer network, like the present peer-to-peer module 228.1, does not comprise the peer-to-peer application 224. Such a peer-to-peer module 228.1 is configured to provide (only) access to the peer-to-peer application 224 e.g. via an API. Each peer-to-peer module (also a node or light node) may comprise a decentral application and at least an API. In the case, the peer-to-peer module is formed as a node the peer-to-peer module (also) comprises at least partly the peer-to-peer application 224. It shall be understood that also the modules 228.1 and 228.2 may be formed as a node of the peer-to-peer network. 333

The peer-to-peer module 228.1 may comprise a communication connection to the sensor module 232 and/or the further source 233. The sensor module 233 and/or the further source 233 may be configured to provide to the peer-to-peer module 228.1 at least one telemetry data set relating to the first entity 202.

The peer-to-peer module 228.1 may provide the at least one telemetry data set comprising at least one parameter (value) to the peer-to-peer application 224 e.g. by transmitting or writing said data to the peer-to-peer application 224. Thereby, the peer-to-peer application 224 is configured such that the received telemetry data set is forwarded to the evaluating means 230 in such a way that no participant 226.1, 226.2, 226.3, 228.1, 228.2 of the peer-to-peer network 222 can read or otherwise access this telemetry data set. By providing said telemetry data set to the peer-to-peer application 224 and thus, to the evaluating means 230, the telemetry data set can be securely evaluated in accordance with the at least one evaluation rule and an evaluation result can be generated.

The at least one evaluation result can be transmitted from the evaluating means 230 by means of the peer-to-peer application 224 to at least the peer-to-peer module 228.2 of the second entity 204. It shall be understood that the evaluation result may also be transmitted to the provider of the analyzed telemetry data set, e.g. the first entity 202.

Based on the evaluation result the receiver of the result, such as the second entity 204, can be configured to initiate or trigger a predefined action. For instance, based on the evaluation result the second entity can generate and send an instructions message preferably by means of the peer-to-peer application 224 to the first entity 202. The instructions message may comprise one or more command for one or more actors to change at least one setting. For instance, the actors can be configured to cause an amendment of at least one telemetry parameter (or vehicle route planning parameter) due to the evaluation result. Another example of a predefined action may be a predefined financial transaction. For instance, the second entity 204 can cause by means of the peer-to-peer application 224 that a particular amount of cryptocurrency is transferred, wherein the amount, sender and/or receiver of this transaction can depend on the generated evaluation result. In other embodiments, the at least one predefined action can also be (automatically) initiated and conducted by the peer-to-peer application 224.

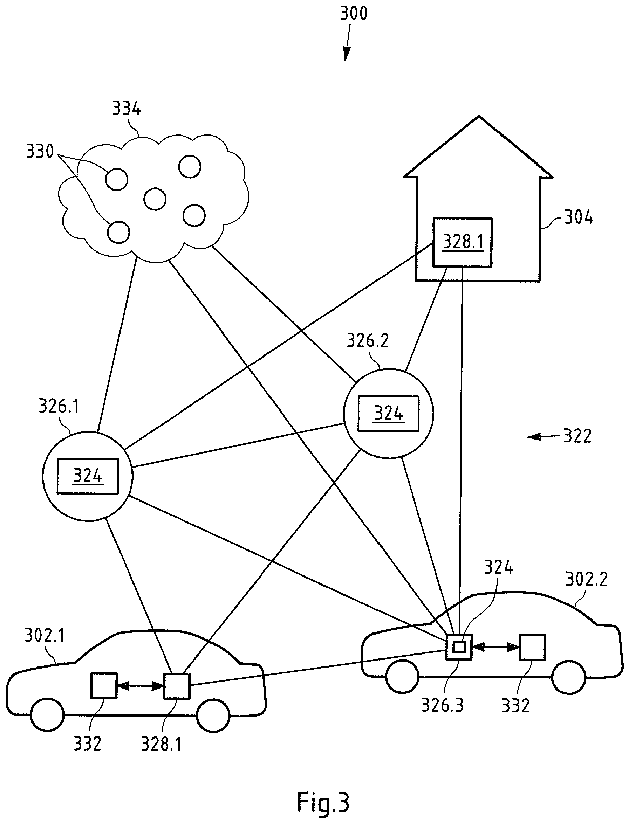

Figure 3 shows a schematic view of a further, more specific, embodiment of a system 300 for securely evaluating telemetry data according to the present invention. In the depicted embodiment, the system 300 comprises two first entities 302.1, 302.2 in form of vehicles 302.1, 302.2. In the present example, it will be assumed that each vehicle 302.1, 302.2 is a car 302.1, 302.2. However, the statements made can be easily transferred to other kinds of entities.

Each car 302.1, 302.2 comprises a sensor module 332 having one or more sensors. By way of example, a sensor can be configured to detect or measure the driven distance per time unit (e.g. km/a), the velocity or speed of the car 302.1, 302.2 (e.g. average speed per preset distance, maximum speed values, etc.), permitted velocity (e.g. by using one or more camera configured to detect speed limit signs), distance to a car driving in front of said car 302.1, 302.2 (e.g. using radar sensor(s)), fuel consumption per time unit or per distance unit, etc. One or more of these or other telemetry parameters can be collected by a collecting means e.g. of the peer-to-peer module 326.3, 328.1 of a car 302.1, 302.2. Further, from one or more of these telemetry parameters, a telemetry data set can then be generated. This telemetry data set can be transmitted by a peer-to-peer module 326.3, 328.1 to the peer-to-peer application 324 e.g. at preset time points.

As can be seen from figure 3, the peer-to-peer module 326.3 of car 302.2 is itself a node 326.3 of the peer-to-peer network 322 while the peer-to-peer module 328.1 is only formed as a participant 328.1.

Furthermore, the depicted system 300 comprises a database 334. In particular, the database 334 is formed as a decentral file storage 334 or decentral database 334 or system 334. The decentral file storage 334 or decentral database 334 is (only) controlled by the peer-to-peer application 324. The decentral file storage 334 or decentral database 334 comprises evaluating means 330 which can be distributed within the decentral file storage 334 or decentral database 334. The data stored in the decentral file storage 334 or decentral database 334 is preferably encrypted. The (encrypting and/or decrypting) keys may be handled and controlled via the peer-to-peer application. Preferably, the (encrypting and/or decrypting) keys are handled by the decentral file storage 334 or decentral database 334. The owner and/or user of a car can be in full control of his data.

It shall be understood that such a system can be used for vehicle data logging, vehicle data analytics, vehicle instrumentation, vehicle re-configuration and emission monitoring with built in data and transactional privacy.

Privacy preserving, secure transactions or execution of computer code comprising the at least one evaluation rule configured to conduct the evaluation of at least one telemetry data set can be achieved with cryptographic tools such as zero knowledge (zk) proofs or zk Succinct Non-interactive Arguments (zk-SNARK).

More particularly, an evaluating means 330 comprising an evaluation rule and/or evaluation algorithm can be separated into two parts: a smart contract on the peer-to-peer application 324 and a private smart contract running on the decentral file storage 334. A privacy preserving protocol, i.e. a protocol message, can be generated and provided to at least the involved entities. The privacy preserving protocol (message) may ensure the privacy of data and the correctness of code execution (SNARK verification is done via the smart contract on chain). The private contract computation can be done by the secured decentral file storage 334.

The peer-to-peer application 324 and/or the evaluating means 330 can be configured such that a received telemetry data set from a car 302.1, 302.2 is deleted, preferably, directly after the evaluation of said data set. For instance, a received telemetry data set can be overwritten e.g. with random numbers or other data. The correctness of the deletion process can be verified by at least a part of the nodes 326.1-326.3 of the peer-to-peer network 322. The peer-to-peer module 326.3, 328 of the provider of said telemetry data set can monitor the deletion process, as well.

The at least one evaluation result can be provided to at least one second entity 304 via a peer-to-peer module 328.2 and e.g. to the provider of the respective telemetry data set, e.g. car 302.1 or 302.2.

Based on the evaluation result the second entity 304, in particular, the peer-to-peer module 328.2 of the second entity 304 can cause that a predefined action or transaction is performed by means of the peer-to-peer application 324.

For instance, the peer-to-peer application 324 may conduct the transfer of a specific amount of cryptocurrency from the provider (e.g. car 302.1 or user of said car 302.1) of the evaluated telemetry data set to the second entity 304. The purpose of the amount may be a car insurance for the car 302.1. The specific amount to be paid may depend on the evaluation result. For instance, the evaluation result may include that a specific threshold (e.g. of a plurality of different preset thresholds) of driven kilometres per year has been increased (without any information about the actual driven kilometres). The actual amount to be transferred may depend on the specific threshold. Alternative or additional criteria may be the detected fuel consumption, aggressive or not aggressive driving style, etc.

It shall be understood that such a system using cryptocurrencies might reduce credit risks for a car insurance provider by including user credit risk and/or payment data into the private contract or by working with deposits and allowing the user to unlock the vehicle only if deposits are paid in advance. Instead of working with deposits a constant stream of 'nano'-payments can be established to pay per use per minimum time unit (e.g. a payment streams that transfers a specific value of digital tokens n accordance to a usage every time unit (e.g. every second)).

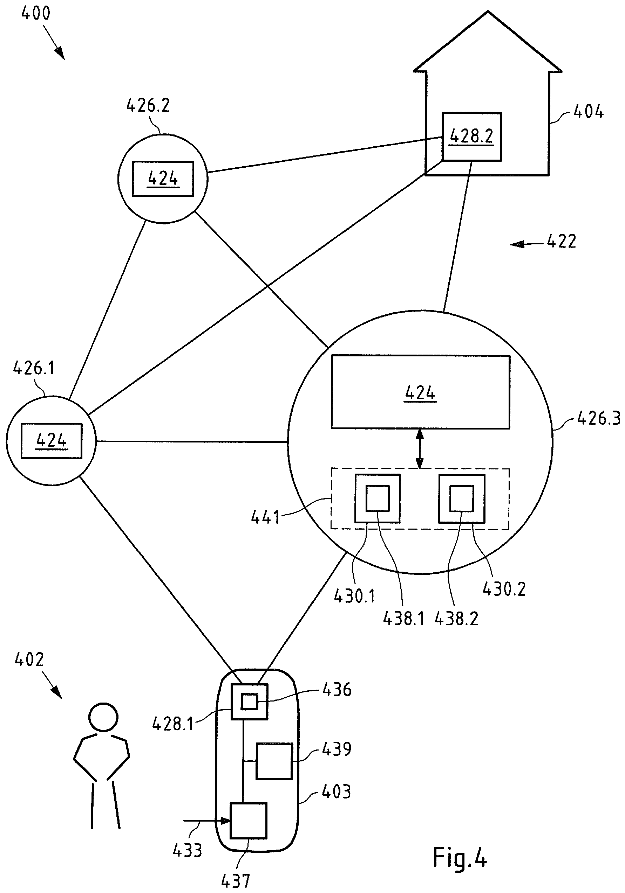

Figure 4 shows a further schematic view of an embodiment of a system 400 according to the present invention. The system 400 comprises an entity 402 (e.g. user), wherein a user terminal 403, such as a mobile phone, tablet computer, notebook, smart card, portable navigation device, etc., is assigned to the entity 402.

It shall be understood that such a device may run an alternative block chain such as IOTA. The peer-to-peer application in system 400 may get the device data via an interface to the IOTA block chain (replication of device data among block chains).

The user terminal 403comprises a communication module 437 configured to receive telemetry data relating to the user of the user terminal 402 from a further source 433. In particular, the telemetry data can be related to the owner of the user terminal 402. The received telemetry data can be securely stored in a storage module 439 of the user terminal 403. In order to transmit a telemetry data set to the peer-to-peer application 424 of the peer-to-peer network 422, the user terminal 402 comprises a peer-to-peer module 428.1. For transmission, as previously described, standard networks and means can be used.

Furthermore, the peer-to-peer module 428.1 comprises an encrypting means 436 configured to encrypt the at least one telemetry data set. Prior to transmitting one or more telemetry data set(s) to the peer-to-peer application 424 the encrypting means 436 may encrypt the telemetry data set. Then, the encrypted telemetry data set can be associated with a unique peer-to-peer identification assigned to the user terminal 402 (and thus, at least indirectly to the owner of the user terminal 402). The encrypted telemetry data set associated with the unique identification can be transmitted to the peer-to-peer application 424.

In the present case, the peer-to-peer network 422 is formed by a plurality of nodes 426.1, 426.2, 426.3, wherein each node 426.1, 426.2, 426.3 comprises the peer-to-peer application 424. The node 426.3 comprises in addition to the peer-to-peer application 424 a plurality of evaluating means 430.1, 430.2 controlled by the peer-to-peer application 424. The node 426.3 may be a particular powerful node 426.3. Powerful means in particular a high computing power.

The evaluating means 430.1, 430.2 may be arranged in a secured environment 441 of the node 426.3. An evaluating means 430.1, 430.2 comprises a decrypting means 438.1, 438.2 configured to decrypt a received encrypted telemetry data set. The peer-to-peer application 424 may be configured to forward a received telemetry data set associated with a peer-to-peer identification to a specific evaluating means 430.1, 430.2 based on said identification. For instance, while a first evaluating means 430.1 may be configured to decrypt and evaluate (only) telemetry data sets associated with an identification I₁, the further evaluating means 430.1 may be configured to decrypt and evaluate (only) telemetry data sets associated with identifications I_{2.1}, I_{2.2}, .etc. The respective evaluating means 430.1, 430.2 decrypts the received telemetry data set by means of the respective decrypting means 438.1, 438.2. A decrypting means may comprise decrypting keys for decrypting the received telemetry data set.

After conducting the evaluation process the evaluation result can be forwarded to the peer-to-peer application 424. The peer-to-peer application 424 may transmit the evaluation result to the peer-to-peer module 428.2 of a second entity 404 and e.g. to the entity 402 or user terminal 403 associated with said entity 402 which has sent said evaluated telemetry data set. Preferably, the evaluation result can be associated with the peer-to-peer identification of the sending entity 402 in order to uniquely assign the evaluation result to the entity 402 to which the telemetry data set belongs. Based on the evaluation result the second entity 404 can initiate a preset action.

In other variants, also the evaluation result may be encrypted. For instance, the evaluating means may also comprise further encrypting means and the receiver of the evaluation results may comprise further corresponding decrypting means.

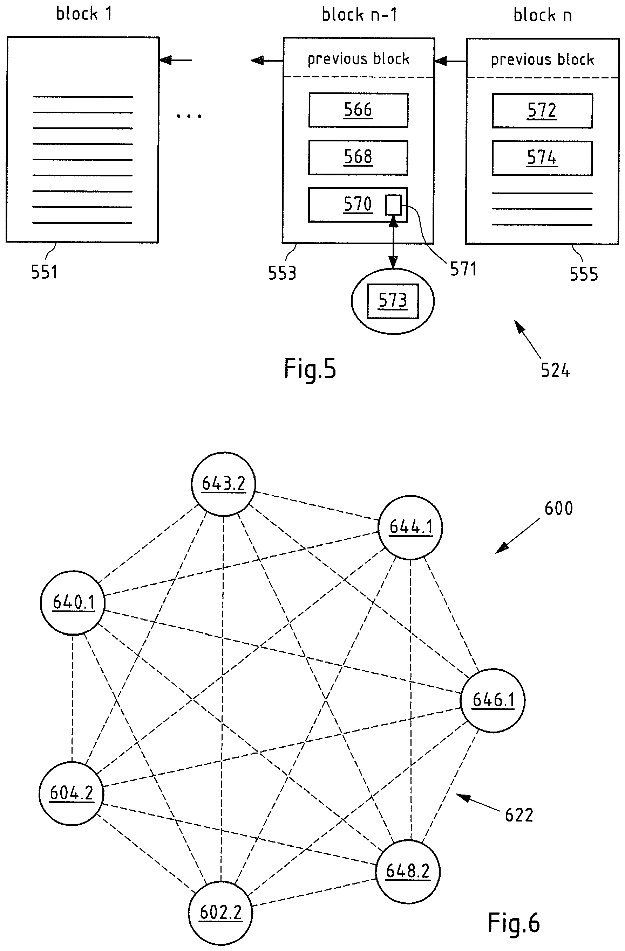

Figure 5 shows a schematic view of an embodiment of a peer-to-peer application 524 according to the present invention.

The depicted peer-to-peer application 524 is a register readable, in particular, by the participants of the peer-to-peer network. Thereby, data e.g. in form of messages can be written and/or read into/from the register 524 by a peer-to-peer module of an unit/entity and/or any other participants in the peer-to-peer network. In a preferred embodiment, the peer-to-peer application 524 may be a block chain 524.

Hereinafter, it is assumed in the following description of the present embodiment that the at least one peer-to-peer application 524 is a block chain 524. However, the following remarks can be easily transferred to other peer-to-peer applications, such as a Directed Acyclic Graph (DAG). A directed acyclic graph, such as IOTA or Tangle, means that blocks (or nodes of the graph) are coupled to each other via directed edges. Thereby, direct means that the (all) edges have (always) a same direction similar to time. In other words, it is not possible to step back. Eventually, acyclic means that loops do not exist.

In alternative embodiments of the peer-to-peer application the block chain can be a permissionless or permissioned block chain. In a specific case the block chain can be public, consortium or private block chain.

In a further embodiment, the peer-to-peer application can be formed with multiple block chains which are connected via mechanisms such as side chains or smart contracts. Interoperability among block chains can be established.

The block chain 524 is formed by at least one block 551, 553, 555, preferably by a plurality of interconnected blocks 551, 553, 555. The first block 551 may also be called genesis block 551. As can be seen, a block 553, 555 (except for the first block 551) refers to each previous block 551, 553. A new block can be created by a computationally intensive process (for example, so called "mining" or through another appropriate process, such as voting) and will be particularly provided to all participants of the peer-to-peer network.

The present block chain 524 is particularly adapted to receive messages from a peer-to-peer module of a previously described entity or from another peer-to-peer device/unit of another participant of the peer-to-peer network. Further, the block chain 524 is particularly adapted to save these messages in the block chain 524. Furthermore, the block chain 524 is configured to generate messages e.g. based on an evaluation process and/or caused by a peer-to-peer module. In particular, the block chain is at least configured to control evaluating means.

In particular, a (newly) received message can be saved and published in the current block 555 of the block chain 524. Due to the configuration of a block chain 524 as a public register 524, said data message of e.g. a peer-to-peer module of an unit can be read by preferably all participants of the peer-to-peer network or data of the message will be stored on decentral file service or distributed block chain database. However, if the message relates to an evaluating means, e.g. a private smart contract or sMPC, and e.g. comprises telemetry data, preferably, a sending peer-to-peer module may ensure by encrypting means that this data is not readable by the participants of the peer-to-peer network. It shall be understood that the peer-to-peer module of the provider of said data set may be configured to read said data set.

As already described, in the present block chain 524 different types of messages and data sets, respectively, for example, within a smart contract (algorithm and/or storage at the block chain 524) can be processed and/or stored. By way of example, the message 570 comprises a generated evaluation transaction agreement.

An example of a generation of such an evaluation transaction agreement 570 will be described in the following:
An evaluation transaction agreement 570 may comprise at least one of the following data:

| | |
|---|---|
| Identification(s): | Peer-to-peer identification(s) assigned to one or more entities involved in the evaluation (e.g. a provider of telemetry data, entity providing the evaluating means, smart oracle used, and receiver of evaluation result) |
| Evaluation rule: | At least one rule/algorithm according to which the evaluation result is generated |
| Prerequisites: | Defined pre-requisites for registering the identification with a peer-to-peer application |
| Terms of input: | Information about the kind of telemetry data to be provided and e.g. about the way in which the telemetry data should be provided and/or where and/or how telemetry data is stored |
| Terms of output: | Rule(s) about the kind of the evaluation result and the output of the evaluation result, e.g. the way in which the evaluation result should be provided |
| Predefined action: | At least one action and/or transaction to be performed depending on the evaluation result (e.g. a first action is performed if a first result is provided and a second action is performed if a second result is provided) |

The predefined action may be a (financial) transaction 572 (e.g. transferring a specific amount of cryptocurrency depending on the evaluation result), a sending action of a specific instruction message 574 comprising instruction(s)/setting(s) (depending on the evaluation result) to an actor e.g. of the entity which provided the evaluated data set, or sending a request message to a user, etc.

The at least one evaluation rule may be an algorithm or the like. For instance, one or more threshold value(s) can be set for one or more telemetry parameter(s). Other more complex algorithms are possible. The at least one evaluation rule may be formed as code executable by an evaluating means.

Further, an evaluation transaction agreement 570 may comprise more data. More information/criteria can be, for example, a time stamp, information about the involved entities, an ID of the transaction and other criteria. Furthermore, an evaluation transaction agreement 570 may cause the creation of an evaluating means or adaption of an already existing evaluating means. By way of example a smart contract 571 comprising means to control a private contract 573 (e.g. installed on a node) can be generated as evaluating means.

An evaluation transaction agreement 570 may be valid for a single conduction of the agreed evaluation process or a particular or unlimited number of different runs of the evaluation process. For instance, the evaluation transaction agreement 570 may be an agreement about the evaluation of telemetry data relating to a patient.

In order to generate an evaluation transaction agreement 570, a peer-to-peer module assigned to an entity, such as a computing device, building, machine, vehicle, user, etc. (e.g. the peer-to-peer module assigned to a car) can exchange evaluation request and response (acceptance) messages via the peer-to-peer application 524. A request message 566 may comprise indications about the above data (identifications, evaluation rule(s), etc.).

In response to a request message 566 an acceptance message 568 can be transmitted by a further entity. An acceptance message 568 may comprise identical or at least similar data details as compared with a request message 566. Additionally, the acceptance message 568 can comprise a reference indication to a previous request, such as the identification of the request message 566.

If, for example, the acceptance message 568 comprises one or more different evaluation rule(s) compared with the rule(s) of the request message 566, the acceptance message 568 can be called a counter-offer message. This can be accepted by the peer-to-peer module of the requester through an acceptance message. Based on this a peer-to-peer module may cause the generation of an evaluation transaction agreement 570.

In particular, there can be multiple request messages and/or accepting messages and/or messages. Each entity can give guidelines, according to which at least one evaluation transaction agreement can be generated. In a preferably automated, such as iterative process, each request message can be associated to an optimally corresponding acceptance message. The block chain 524 may also be configured to generate, based on the messages of a peer-to-peer module an evaluation transaction agreement 570.

An evaluation transaction agreement 570 may be stored within a smart contract and/or private contract in a block 553. A smart contract 570 may comprise computer program code. Based on the evaluation transaction agreement 570 an evaluating means can be (newly) generated or respectively configured, as previously described. The evaluating means can be configured such that it comprises secret decrypting key configured to decrypt telemetry data sets associated with peer-to-peer identification(s), as agreed in the evaluation transaction agreement 570.

In an alternative embodiment a private contract computation can be used in combination with a measured launch environment or a secure hardware enclave for attestation and/or sealing. A telemetry data set may be stored and decrypted in the enclave which is directly controlled by the peer-to-peer application. After the evaluation process is done the provided telemetry data set may be overwritten in the memory by e.g. random numbers or other data. By using zk-Proof Systems and/or trusted computing hardware it can be checked that the data are erased in the device to prevent fraud. In the case of sMPC the data may be distributed among several entities and encryption may be used to ensure that the data are not stored in a meaningful format in an entity.

Messages and amendments of messages can be verified by the peer-to-peer network, in particular, the participants of the peer-to-peer network.

In particular, the peer-to-peer application 524 is configured to save the messages 566 to 574 in a tamper-proof manner. This is done essentially by the fact that through the entire peer-to-peer network, for example, an agreement or message or status parameter or the like can be verified by the cumulative calculation power of the entire peer-to-peer network.

Preferably, at least the above described messages, such as the agreements and other messages, can be hashed together in pairs in a block of the block chain by a Merkle tree. In particular, only the last hash value, the so-called root hash, is noted as a checksum in the header of a block. Then, the block can be coupled with the previous block. Chaining of the blocks can be performed using this root hashes. Each block can include the hash of the entire previous block header in its header. This makes it possible to clearly define the order of the blocks. In addition, this may also prevent the subsequent modification of previous blocks and the messages stored in the previous blocks, since, in particular, the hashes of all subsequent blocks would have to be recalculated in a short time.

In addition, data feeds can be provided by the peer-to-peer application (so called smart oracles.).

Figure 6 shows a schematic view of another embodiment of a system 600 of the invention. In the present embodiment only nodes and participants 602.2 to 648.2 of the peer-to-peer network 622 are shown. In the present example, it is assumed that all nodes 602.2 to 648.2 comprise the peer-to-peer application (not shown).

The nodes 602.2, 640.1, 643.2, 648.2 may be first entities, such as cars, and their respective peer-to-peer modules, respectively. The node 604.2 may be realized by a peer-to-peer module of a second entity. Nodes 644.1 and 646.1 may be particular powerful nodes each comprising a secured environment with one or more evaluating means. It shall be understood that nodes can be full, remote or light nodes.

As can be seen, two different types of peers or node computers 640.1, 644.1, 646.1 and 602.2, 604.2, 643.2, 648.2 are presently illustrated. All peers 640.1, 644.1, 646.1 and 602.2, 604.2, 643.2, 648.2 are comprised by the peer-to-peer network 622. In the present embodiment, however, only a part of the peers 640.1, 644.1, 646.1 and 602.2, 604.2, 643.2, 648.2 in the present case, the peers 640.1, 644.1, 646.1, check the validity of e.g. an evaluation process and/or further data stored in the peer-to-peer application messages, such as agreements, request messages, and the like.

Furthermore, only a part of the entire peers can be configured to store the peer-to-peer application and/or only a part of the peers can be configured to execute the algorithms of a smart contract and/or private contract. Since the validation/verification of e.g. identification data requires a considerable computational effort, it may be advantageous for reasons of efficiency, if only a part of the peers 640.1, 644.1, 646.1, especially particularly powerful peers 640.1, 644.1, 646.1, perform the validation and/or evaluation algorithms.

Validation and optimization can be done on-chain or off-chain. Off-chain validation and/or optimization can be managed by the peer-to-peer application, like the code on the block chain. Powerful means in particular a high computing power. In other words, in the present case a valid entry in the peer-to-peer application, such as a block chain, is assumed if (only) a part of the peers 640.1, 644.1, 646.1 comes to a positive result. It shall be understood that only a single, especially particularly powerful peer can perform the validation and/or optimization process.

Similarly, in an alternative (not shown) embodiment, a particularly large peer-to-peer network may be divided in two or more clusters. In a corresponding peer-to-peer network, for example, a validation will only be carried out by the members of one cluster (e.g. sharing of a block chain to improve the scalability). In a further embodiment the peer-to-peer application can be formed using multiple block chains. These block chains are connected via frameworks such as sidechains.

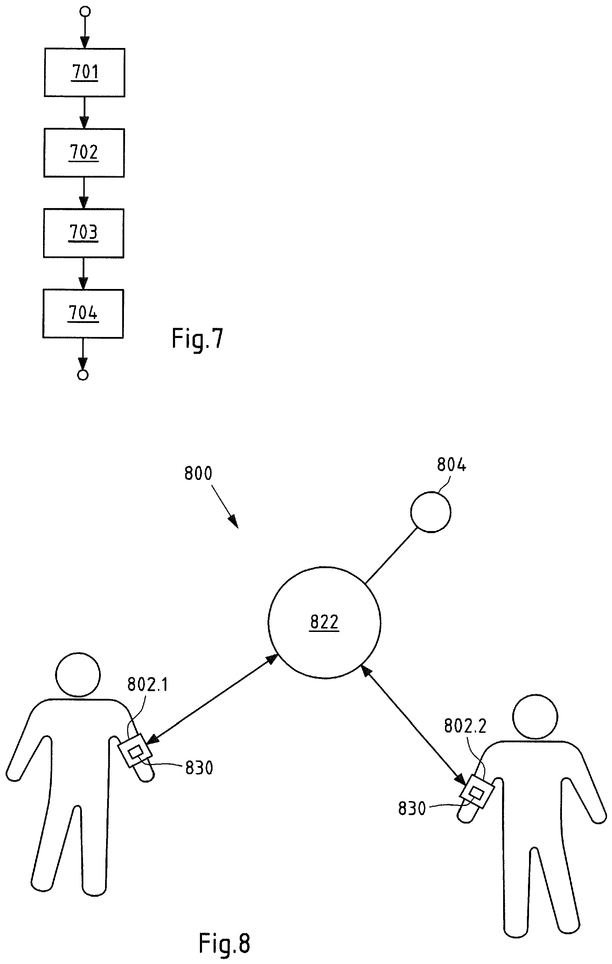

Figure 7 shows a diagram of a first embodiment of a method according to the present invention.

In a first step 701, at least one telemetry data set relating to at least one first entity can be transmitted from a peer-to-peer module assigned to the first entity to at least one peer-to-peer application of at least one peer-to-peer network. As described hereinbefore, prior to transmitting the telemetry data set the respective data can be detected, collected and/or encrypted.

In a next step 702, an evaluation process of the transmitted telemetry data set is controlled by the peer-to-peer application. This means that a received telemetry data set may be forwarded by the peer-to-peer application to the correct evaluating means e.g. based an analysis of a peer-to-peer identification associated with the received telemetry data set and a previously generated evaluation transaction agreement. For instance, the peer-to-peer application can compare the received peer-to-peer identification with the peer-to-peer identification(s) of one or more stored evaluation transaction agreement(s).

Thereby, each evaluation transaction agreements may comprise a (unique) identification of the evaluating means to be used. During the conduction of the evaluation process, the peer-to-peer application and at least a part of the nodes of the peer-to-peer network, respectively, monitor whether unauthorized units try to access the telemetry data or the like. Further, it is monitored that the evaluating means does not output the telemetry data or the like to an unauthorized unit.

After the evaluation process, in a further step 703, at least one evaluation result is provided by the peer-to-peer application to at least one second entity without providing the received telemetry data set. Further, cryptographic proofs of the evaluation process may be validated by the peer-to-peer application. Preferably, the evaluating means only provides an evaluation result (in accordance with the terms of output of an agreement) to the peer-to-peer application. The peer-to peer application may transmit said evaluation result based on the information stored in an evaluation transaction agreement to the one or more receiver(s) of the evaluation result. Further, the peer-to-peer application may be configured to control the irretrievable deletion of the at least one evaluated telemetry data set.

Furthermore, in step 704, the peer-to-peer application may perform at least one predefined action. Based on the evaluation result, the peer-to-peer application may - automatically and/or triggered by a peer-to-peer module of a receiver (e.g. second entity) of the evaluation result - perform the at least one predefined action, such as a financial transaction and/or control action, such as providing one or more actor(s) with one or more amended set value(s) and/or a user request message asking for data disclosure while receiving a payment.

Figure 8 shows a schematic view of a further embodiment of a system 800 for securely evaluating telemetry data relating to a user according to the present invention.

As can be seen from figure 8, a user can comprise a medtech device 802.1, 802.2 in form of a wearable device 802.1, 802.2. Each medtech device 802.1, 802.2 may comprise a sensor module 830. A sensor module 830 may comprise one or more sensor(s) configured to measure one or more telemetry parameter(s) in form of body parameter(s) of a user and patient, respectively. In other words, at least one telemetry parameter relating to a first entity (patient or user of the medtech device 802.1, 802.2 or the medtech device 802.1, 802.2 may be the first entity 802.1, 802.2, wherein the telemetry parameter relates to the medtech device 802.1, 802.2, and thus, (indirectly) to the patient assigned to the medtech device 802.1, 802.2 and/or using said medtech device 802.1, 802.2). By way of example, the medtech device 802.1, 802.2 may be configured to measure the heart rate, the blood pressure or the like, by using said sensor module 830. It shall be understood that the communication between sensor module 830 (which might also be one or more remote sensor(s)) and the medtech device can be secured by using at least one singing module inside a sensor (for hashing, signing, time-stamping and / or encrypting data). A peer-to-peer communication protocol (such as Whisper) can be used to establish direct communication with a sensor. It shall be understood that a similar approach can be used to establish secure communication with an actor (e.g. insulin pump).

Each medtech device 802.1, 802.2 may comprise a (not shown) peer-to-peer module. The peer-to-peer module may transmit at least one measured body parameter to a (not shown) peer-to-peer application of a peer-to-peer network 822. As previously described, the peer-to-peer application is configured to securely control the conduction of an evaluation process (e.g. orchestrated by smart and private contracts; using secured environment or sMPC). The at least one evaluation result of the evaluation process can be forwarded to a second entity 804, as described hereinbefore.

Such a process may be conducted in accordance with a previously generated evaluation transaction agreement about the evaluation of telemetry data relating to a patient. In such an agreement, a criterion can be generated which must be fulfilled in order to provide the at least one telemetry parameter. For instance, the criterion can be a specific amount of cryptocurrency which has to be transferred from e.g. a second entity to the first entity (e.g. patient).

For example, the at least one medtech device 802.1, 802.2 can (continuously) measure at least one body parameter during the conduction of e.g. a specific treatment, like taking a specific medication e.g. for a specific disease. The healing effect and/or one or more side effects of the specific medication can be evaluated in case the medtech device 802.1, 802.2 transmits one or more body parameter and/or other parameter(s) to the peer-to-peer application for evaluation. Besides one or more body parameter measured by the medtech device 802.1, 802.2 further telemetry parameter(s), such as taking a specific dosage of said specific medication at a specific time, can be transmitted to the peer-to-peer application. For instance, a medtech device 802.1, 802.2 can comprise a user interface for inputting said at least one further telemetry parameter. Alternatively or additionally, e.g. a user terminal assigned to the patient and comprising a peer-to-peer module can be used. Additional telemetry data e.g. provided by additional medtech devices, such as mobile or stationary device(s), may be provided to the peer-to-peer application for evaluation.
In the case, a medtech device 802.1, 802.2 is not uniquely assigned to a specific user, the medtech device 802.1, 802.2 may comprise an interface for receiving a (unique) peer-to-peer identification e.g. from a user terminal uniquely assigned to the patient (e.g. a smart phone, smart card or the like). Preferably, said user terminal, such as a smart card, may comprise a peer-to-peer module. In this case, the user terminal can forward the unique peer-to-peer identification e.g. provided from the peer-to-peer application via the peer-to-peer module to the medtech device 802.1, 802.2. Then the medtech device 802.1, 802.2 may transmit (by means of a peer-to-peer module) the at least one telemetry parameter relating to said patient in a secure manner (e.g. as described before) using said unique peer-to-peer identification of the patient to the peer-to-peer application. For instance, the medtech device 802.1, 802.2 may be a (stationary) x-ray device, a (stationary) ultrasonic device, a nuclear medicine, a DNA sequencing/slicing, a body probe or sample analysis device (e.g. Cardiology Analytics, Nephrology Analytics, Tissue analytics, Blood Analytics, Breath Analytics), or the like.

The medtech device (which might not uniquely assigned to a specific user) may have a secured environment. A user may deploy an user identification, personal data, code and/or cryptographic key(s) (or parameter(s) to generate (new) cryptographic key(s)) into the secured environment of the medtech device. Such a deployment process can be started by providing a personal identification, transaction address and/or a hash address to the device. This can be done by reading out an identification, address or hash code of the medtech device (e.g. via QR code or RFID) with a mobile unit (e.g. a smart phone) of a user and sending a deployment request to the medtech device (or in a manual process of typing in a device ID/address). The medtech device may send a (signed) confirmation to the user upon successful completion of the deployment process. It shall be understood that such a deployment process can also be initiated from a medtech device (e.g. medtech device has a user terminal to enter the user address of to be deployed data/code and/or the medtech device might be able to read out an identification of another device; e.g. wearable, body implant, smart phone, QR code or RFID). In a further embodiment the medtech device may be connected with a (decentral) database with (pre-configured) user identification(s) and the user can select its identification (pseudonym) and can confirm its identification by providing e.g. authentication data (e.g. one or more factor authentication).

After successful completion of the deployment process the secured environment can read out (encrypted) sensor data, (decrypt and) process the sensor data and/or store (encrypted) data in a secured database assigned (only) to the user ('User Controlled Patient's Records'). The user would have full control about its data by having access to the cryptographic keys that might have been exchanged between the medtech device and the peer-to-peer application. The user might decide to provide selective access to the data to other entities. After successful storage of the user data in said database the medtech device my delete all user data stored in the secured environment. The medtech device can either send a confirmation about the deletion process or a zk-Proof-System is used to allow the user to validate that the data were deleted.

In addition, the one or more telemetry parameter(s) and/or evaluation result(s) of said parameter (s) relating to a patient can be stored in a secured database (e.g. IPFS, BigchainDB or cloud DB) which is only controllable by the patient. At least part of the nodes of the peer-to-peer network 822 may be configured to monitor the database, e.g. the access to said database.

It shall be understood that the data stored in the secured database might only be accessed by the patient.

In shall be understood that further devices can be integrated into this system following the procedures described below. For instance, Point of Sales terminals can be integrated to combine user data with sales data (eg which prescriptions was bought when and where). Other examples are terminals or systems of medical practitioner or hospitals or insurance companies. A user can decide to which entity access to personal data is granted,

Storing user data (in a herein described secure way) in a (decentral) record (of a peer-to-peer application) may result in 'User Controlled Patient's Records'. Data stored in such a system can be any kind of patient related data including master and/or diagnosis data (e.g. age, gender, race, abnormalities, distinctive features, disease, medical conditions, anamneses, analysis / diagnosis data, diagnosis findings, Prescriptions, use of medications).

As a further aspect of the invention a user can decide to share data with a system of smart and private contracts (or sMPC) for secure processing of its personal data. A third party might have set up such a system to process for instance DNA data and e.g. analyze the data with powerful (off-chain) algorithm(s). According to a preferred embodiment, said system can include machine learning means and/or artificial intelligence means. One or more algorithm(s) may process the personal user data in a secure environment to generate a specific output agreed between user and third party prior to processing.

Examples for such an output are:
a) Algorithm analyzing patient DNA data without disclosing the DNA data to the third party; output provides an 'algorithmic evidence' that the DNA data can be valuable for the third party (which can be a pharmaceutical company). The third party can then contact a patient to request disclosure of the DNA data upon entering into a (smart) contract agreement with the patient. Contract terms can be one-time payment terms or payments terms linked to an economic success of a drug and / or treatment development. Payments can be done in a secure way by the use of cryptocurrencies. It shall be understood that a patient might be unknown to the third party by using a system described above, but can still receive a request message from the third party. Or the third party just sends a quote to a patient for disclosing the DNA data. Upon agreement by the patient the DNA data will be (automatically) disclosed.
b) The system may support a process in which a patient wants to analyze his or her DNA data to identify health risks. A third party might have set up a (paid) system to analyze the personal DNA in a secure environment without having access to the personal DNA data. The system generates an (encrypted) output about the health risks. The output might just be decrypted by the patient.
c) The system may support setting emergency alarms and/or controlling actors to apply medications in accordance to a schedule or health conditions (e.g. insulin pump, heart pacer). Patient data may be (continuously) analyzed by a secure environment. In case of abnormal or critical conditions emergency alarms can be generated, send to pre-defined recipients and/or actors triggered.
d) A further application might be health insurance contracts providing a premium as an output. Other personal data are not shared with the insurance company.

By using zk-Proofs a patient and third party can validate that the processing with done in accordance to terms defined in advance.

In a further embodiment a (encrypted, decentral) secure 'Multi-Patient Data Management' may be created by storing patient data (e.g. into a Database or by storing addresses of 'User Controlled Patient's Records' or by storing anonymous patient data). An algorithm and/or a smart contract may search for specific patient data patterns (e.g. age, gender, DNA defects, specific diseases). The algorithm and/or a smart contract may identify relevant (anonymous) patients. The algorithm can run in a secured environment. A third party can then trigger the secure environment to contact the patient and ask him or her to participate in a campaign or disclose detailed data.

Such a system can be used by pharmaceutical or insurance companies for recruiting the patients with specific patterns (e.g. for a clinical testing or drug effectiveness testing campaign). Financial incentives can be agreed upon in (smart) contracts and paid in cryptocurrencies (depending on commercial success).

Data of the 'Multi-Patient Data Management' system can again be stored either on IPFS, BigchainDB or a Cloud. It shall be understood that such a system for 'Multi-Patient Data Management' can be generalized beyond patient data. In case of vehicles/machines such as system can be a secure system for vehicle fleet data management.

In a further embodiment such a system can be used for secure 'Treatment Life-Cycle Analysis and Management'. A patient agrees to provide (selective) access and/or transparency on its 'User Controlled Patient's Records' to a third party. Conditions for such an access can be laid out in a smart/private contract. Further data can be included in the (decentral) patient record, e.g. medical practitioner data, patient's self-observations, use of biomarkers and analytical results, hospital stays, doctor appointments, other point of care touch-points. The data might only be disclosed to a secure environment.

A third party can use secured environment data processing for instance to analyze the effectiveness of drugs/prescriptions on a patient including perceived health conditions, healing, side effects, interactions with other physical conditions, usage of a drug / prescriptions, etc. A pharmaceutical or insurance company can do a proper clinical testing or drug effectiveness testing campaign.

In a further application medical practitioners can for example be paid based on success of healing or an insurance company can increase a health premium if patient does not take drugs / prescriptions without having access to the detailed patient data.

## Claims

1. System (200, 300, 400, 600, 800) for evaluating telemetry data, comprising:
- at least one peer-to-peer module (228.1, 328.1, 326.3, 428.1) assigned to at least one first entity (202, 302.1, 302.2, 402, 602.2, 640.1, 643.2, 648.2, 802.1, 802.2),
- at least one peer-to-peer network (222, 322, 422, 622, 822) with at least one peer-to-peer application (224, 324, 424, 524),
- wherein the peer-to-peer module (228.1, 328.1, 326.3, 428.1) is configured to transmit at least one telemetry data set relating to the first entity (202, 302.1, 302.2, 402, 602.2, 640.1, 643.2, 648.2, 802.1, 802.2) to the peer-to-peer application (224, 324, 424, 524),
- wherein the peer-to-peer application (224, 324, 424, 524) is at least configured to control evaluating means (230, 330, 430.1, 430.2) configured to evaluate the at least one telemetry data set according to at least one preset evaluation rule,
- wherein the peer-to-peer application (224, 324, 424, 524) is configured to control the evaluating means (230, 330, 430.1, 430.2) such that at least one evaluation result is provided to at least one second entity (204, 304, 404, 604.2, 804) without providing the received telemetry data set,
- wherein the peer-to-peer application (224, 324, 424, 524) and at least a part of computer nodes (226.1, 226.2, 226.3, 326.1, 326.2, 426.1, 426.2) of the peer to peer network (222, 322, 422, 622, 822) monitor whether unauthorized units try to access the telemetry data, and
- wherein, based on the monitoring result, the peer-to-peer application (224, 324, 424, 524) may perform at least one predefined action.

2. System (200, 300, 400, 600, 800) according to claim 1, **characterized in that**
- the evaluating means (230, 330, 430.1, 430.2) is further configured to generate at least one protocol message comprising protocol data about the conducted evaluation process,
- wherein the peer-to-peer application (224, 324, 424, 524) is configured to provide the protocol message to at least one of the first entity (202, 302.1, 302.2, 402, 602.2, 640.1, 643.2, 648.2, 802.1, 802.2) and the second entity (204, 304, 404, 604.2, 804).

3. System (200, 300, 400, 600, 800) according to claim 1 or 2, **characterized in that** at least one peer-to-peer module (228.1, 228.2, 328.1, 326.3, 328.2, 428.1, 428.2) assigned to at least one of the first entity (202, 302.1, 302.2, 402, 602.2, 640.1, 643.2, 648.2, 802.1, 802.2) and the second entity (204, 304, 404, 604.2, 804) is configured to cause generating of at least one evaluation transaction agreement about at least the terms for input of the telemetry data set and/or the at least one evaluation rule and/or terms for output of the evaluation result.

4. System (200, 300, 400, 600, 800) according to any of the preceding claims, **characterized in that**
- the peer-to-peer application (224, 324, 424, 524) comprises the evaluating means (230, 330, 430.1, 430.2),
and/or
- at least one database (334) controllable by the peer-to-peer application (224, 324, 424, 524) comprises the evaluating means (230, 330, 430.1, 430.2), and/or
- at least one computer node (426.3) of the peer-to-peer network (222, 322, 422, 622) comprises the evaluating means (230, 330, 430.1, 430.2).

5. System (200, 300, 400, 600, 800) according to any of the preceding claims, **characterized in that**
- the at least one telemetry data set relating to the first entity (202, 302.1, 302.2, 402, 602.2, 640.1, 643.2, 648.2, 802.1, 802.2) is associated with a unique peer-to-peer identification of the first entity (202, 302.1, 302.2,402, 602.2, 640.1, 643.2, 648.2, 802.1, 802.2), and
- wherein the at least one evaluation result is associated with the unique peer-to-peer identification of the first entity (202, 302.1, 302.2, 402, 602.2, 640.1, 643.2, 648.2, 802.1, 802.2).

6. System (200, 300, 400, 600, 800) according to claim 5, **characterized in that**
- the peer-to-peer module (228.1, 328.1, 326.3,428.1) assigned to the first entity (202, 302.1, 302.2, 402, 602.2, 640.1, 643.2, 648.2, 802.1, 802.2) comprises encrypting means (436) configured to encrypt the at least one telemetry data set,
- wherein the peer-to-peer module (228.1, 328.1, 326.3, 428.1) assigned to the first entity (202, 302.1, 302.2, 402, 602.2, 640.1, 643.2, 648.2, 802.1, 802.2) is configured to transmit the encrypted telemetry data set together with the peer-to-peer identification of the first entity (202, 302.1, 302.2, 402, 602.2, 640.1, 643.2, 648.2, 802.1, 802.2) to the peer-to-peer application (224, 324, 424, 524).
- wherein the peer-to-peer application (224, 324, 424, 524) is configured to forward the received encrypted telemetry data set to the evaluating means (230, 330, 430.1, 430.2) based on the peer-to-peer identification associated with the received telemetry data set,
- wherein the evaluating means (230, 330, 430.1, 430.2) comprises at least one decryption means (438.1, 438.2) for decrypting the forwarded telemetry data set.

7. System (200, 300, 400, 600, 800) according to any of the preceding claims, **characterized in that**
- the at least one first entity (202, 302.1, 302.2, 402, 602.2, 640.1, 643.2, 648.2, 802.1, 802.2) is a vehicle (202, 302.1, 302.2, 402, 602.2, 640.1, 643.2, 648.2, 802.1, 802.2) comprising at least one sensor module (332) configured to detect at least one telemetry parameter relating to the vehicle (202, 302.1, 302.2, 402, 602.2, 640.1, 643.2, 648.2, 802.1, 802.2),
- wherein the detected telemetry parameter is provided to the peer-to-peer module (228.1, 328.1, 326.3, 428.1) assigned to the vehicle (202, 302.1, 302.2, 402, 602.2, 640.1, 643.2, 648.2, 802.1, 802.2).

8. System (200, 300, 400, 600, 800) according to any of the preceding claims, **characterized in that**
- the at least one first entity (202, 302.1, 302.2, 402, 602.2, 640.1, 643.2, 648.2) is a medical technology device (202, 302.1, 302.2, 402, 602.2, 640.1, 643.2, 648.2, 802.1, 802.2) comprising at least one sensor module (332) configured to measure at least one telemetry parameter relating to at least one user of the medical technology device (202, 302.1, 302.2, 402, 602.2, 640.1, 643.2, 648.2, 802.1, 802.2),
- wherein the measured telemetry parameter is provided to the peer-to-peer module (228.1, 328.1, 326.3, 428.1) assigned to the medical technology device (202, 302.1, 302.2, 402, 602.2, 640.1, 643.2, 648.2, 802.1, 802.2).

9. System (200, 300, 400, 600, 800) according to any of the preceding claims, **characterized in that**
- the at least one peer-to-peer application (224, 324, 424, 524) is a decentralized register or a shared database,
- wherein the peer-to-peer application (224, 324, 424, 524) is configured to store data with given certain proofs or signatures.

10. System (200, 300, 400, 600, 800) according to any of the preceding claims, **characterized in that** the at least one peer-to-peer application (224, 324, 424, 524) is a block chain or decentral ledger comprising at least two blocks (551, 553, 555) coupled to each other.

11. System (200, 300, 400, 600, 800) according to one of the preceding claims, **characterized in that** at least a part of the computer nodes (226.1, 226.2, 226.3, 326.1, 326.2, 326.3, 426.1, 426.2, 426.3) and/or participants (228.1, 228.2, 328.1, 328.2, 428.1, 428.2) of the peer-to-peer network (222, 322, 422, 622, 822) is/are configured to validate at least the correctness of at least one conducted evaluation process.

12. Method for evaluating at least one telemetry data set, comprising:
- transmitting at least one telemetry data set relating to at least one first entity (202, 302.1, 302.2, 402, 602.2, 640.1, 643.2, 648.2, 802.1, 802.2) from a peer-to-peer module (228.1, 328.1, 326.3,428.1) assigned to the first entity (202, 302.1, 302.2, 402, 602.2, 640.1, 643.2, 648.2, 802.1, 802.2) to at least one peer-to-peer application (224, 324, 424, 524) of at least one peer-to-peer network (222, 322, 422, 622, 822),
- at least controlling an evaluation process of the telemetry data set by the peer-to-peer application (224, 324, 424, 524), and
- providing at least one evaluation result by the peer-to-peer application (224, 324, 424, 524) to at least one second entity (204, 304, 404, 604.2, 804) without providing the received telemetry data set,
- wherein the peer-to-peer application (224, 324, 424, 524) and at least a part of computer nodes (226.1, 226.2, 226.3, 326.1, 326.2, 426.1, 426.2) of the peer to peer network (222, 322, 422, 622, 822) monitor whether unauthorized units try to access the telemetry data, and
- wherein, based on the monitoring result, the peer-to-peer application (224, 324, 424, 524) may perform at least one predefined action.

13. Peer-to-peer application (224, 324, 424, 524), comprising:
- means for receiving at least one telemetry data set relating to at least one first entity (202, 302.1, 302.2, 402, 602.2, 640.1, 643.2, 648.2, 802.1, 802.2),
- means for controlling evaluating means (230, 330, 430.1, 430.2) configured to evaluate the at least one telemetry data set according to at least one preset evaluation rule, and
- means for providing at least one evaluation result to at least one second entity (204, 304, 404, 604.2, 804) without providing the received telemetry data set,
- wherein the peer-to-peer application (224, 324, 424, 524) and at least a part of computer nodes (226.1, 226.2, 226.3, 326.1, 326.2, 426.1, 426.2) of the peer to peer network (222, 322, 422, 622, 822) monitor whether unauthorized units try to access the telemetry data, and
- wherein, based on the monitoring result, the peer-to-peer application (224, 324, 424, 524) may perform at least one predefined action.

14. Peer-to-peer module (228.1, 328.1, 326.3, 428.1) assignable to at least one first entity (202, 302.1, 302.2, 402, 602.2, 640.1, 643.2, 648.2, 802.1, 802.2), comprising:
- at least one input means configured to receive at least one telemetry data set relating to the first entity (202, 302.1, 302.2, 402, 602.2, 640.1, 643.2, 648.2, 802.1, 802.2),
- at least one encrypting means (438.1, 438.2) configured to encrypt the received telemetry data set, and
- at least one communicating means configured to transmit the encrypted telemetry data set together with a unique peer-to-peer identification assigned to the first entity (202, 302.1, 302.2, 402, 602.2, 640.1, 643.2, 648.2, 802.1, 802.2) to at least one peer-to-peer application (224, 324, 424, 524) of at least one peer-to-peer network (222, 322, 422, 622, 822) such that the peer-to-peer application (224, 324, 424, 524) and at least a part of computer nodes (226.1, 226.2, 226.3, 326.1, 326.2,426.1, 426.2) of the peer to peer network (222, 322, 422, 622. 822) monitor whether unauthorized units try to access the telemetry data, and
- wherein, based on the monitoring result, the peer-to-peer application (224, 324, 424, 524) may perform at least one predefined action.

15. A medical technology device (802.1, 802.2) comprising at least one peer-to-peer module (228.1, 328.1, 326.3, 428.1) according to claim 14.

## Patentansprüche

1. System (200, 300, 400, 600, 800) zum Auswerten von Telemetriedaten, umfassend:
- mindestens ein Peer-to-Peer-Modul (228.1, 328.1, 326.3, 428.1), das mindestens einer ersten Entität (202, 302.1, 302.2, 402, 602.2, 640.1, 643.2, 648.2, 802.1, 802.2) zugeordnet ist,
- mindestens ein Peer-to-Peer-Netzwerk (222, 322, 422, 622, 822) mit mindestens einer Peer-to-Peer-Anwendung (224, 324, 424, 524),
- wobei das Peer-to-Peer-Modul (228.1, 328.1, 326.3, 428.1) eingerichtet ist zum Übertragen mindestens eines Telemetriedatensatzes, der sich auf die erste Entität (202, 302.1, 302.2, 402, 602.2, 640.1, 643.2, 648.2, 802.1, 802.2) bezieht, an die Peer-to-Peer-Anwendung (224, 324, 424, 524),
- wobei die Peer-to-Peer-Anwendung (224, 324, 424, 524) zumindest eingerichtet ist zum Steuern eines Auswertemittels (230, 330, 430.1, 430.2), das so eingerichtet ist, dass der mindestens eine Telemetriedatensatz gemäß mindestens einer vordefinierten Auswerteregel ausgewertet wird,
- wobei die Peer-to-Peer-Anwendung (224, 324, 424, 524) eingerichtet ist zum Steuern des Auswertemittels (230, 330, 430.1, 430.2) derart, dass mindestens ein Auswerteergebnis an mindestens eine zweite Entität (204, 304, 404, 604.2, 804) bereitgestellt wird, ohne den empfangenen Telemetriedatensatz bereitzustellen,
- wobei die Peer-to-Peer-Anwendung (224, 324, 424, 524) und mindestens ein Teil der Computerknoten (226.1, 226.2, 226.3, 326.1, 326.2, 426.1, 426.2) des Peer-to-Peer-Netzwerks (222, 322, 422, 622, 822) überwachen, ob unberechtigte Einheiten versuchen, auf die Telemetriedaten zuzugreifen, und
- wobei die Peer-to-Peer-Anwendung (224, 324, 424, 524) auf der Grundlage des Überwachungsergebnisses mindestens eine vordefinierte Aktion durchführen kann.

2. System (200, 300, 400, 600, 800) nach Anspruch 1, **dadurch gekennzeichnet, dass**
- das Auswertemittel (230, 330, 430.1, 430.2) ferner so eingerichtet ist, dass mindestens eine Protokollnachricht erzeugt wird, die Protokolldaten über den durchgeführten Auswertungsprozess enthält,
- wobei die Peer-to-Peer-Anwendung (224, 324, 424, 524) so eingerichtet ist, dass die Protokollnachricht der ersten Entität (202, 302.1, 302.2, 402, 602.2, 640.1, 643.2, 648.2, 802.1, 802.2) und/oder der zweiten Entität (204, 304, 404, 604.2, 804) bereitgestellt wird.

3. System (200, 300, 400, 600, 800) nach Anspruch 1 oder 2, **dadurch gekennzeichnet, dass** mindestens ein Peer-to-Peer-Modul (228.1, 228.2, 328.1, 326.3, 328.2, 428.1, 428.2), das der ersten Entität (202, 302.1, 302.2, 402, 602.2, 640.1, 643.2, 648.2, 802.1, 802.2) und/oder der zweiten Entität (204, 304, 404, 604.2, 804) zugeordnet ist, so eingerichtet ist, dass ein Generieren mindestens einer Auswertetransaktionsvereinbarung über zumindest die Bedingungen für die Eingabe des Telemetriedatensatzes und/oder die mindestens eine Auswerteregel und/oder die Bedingungen für die Ausgabe des Auswerteergebnisses bewirkt wird.

4. System (200, 300, 400, 600, 800) nach einem der vorherigen Ansprüche,
**dadurch gekennzeichnet, dass**
- die Peer-to-Peer-Anwendung (224, 324, 424, 524) das Auswertemittel (230, 330, 430.1, 430.2) umfasst,
und/oder
- mindestens eine durch die Peer-to-Peer-Anwendung (224, 324, 424, 524) steuerbare Datenbank (334) das Auswertemittel (230, 330, 430.1, 430.2) umfasst,
und/oder
- mindestens ein Computerknoten (426.3) des Peer-to-Peer-Netzes (222, 322, 422, 622) das Auswertemittel (230, 330, 430.1, 430.2) umfasst.

5. System (200, 300, 400, 600, 800) nach einem der vorherigen Ansprüche,
**dadurch gekennzeichnet, dass**
- der mindestens eine Telemetriedatensatz, der sich auf die erste Entität (202, 302.1, 302.2, 402, 602.2, 640.1, 643.2, 648.2, 802.1, 802.2) bezieht, mit einer eindeutigen Peer-to-Peer-Identifikation der ersten Entität (202, 302.1, 302.2, 402, 602.2, 640.1, 643.2, 648.2, 802.1, 802.2) assoziiert ist, und
- wobei das mindestens eine Auswerteergebnis mit der eindeutigen Peer-to-Peer-Identifikation der ersten Entität (202, 302.1, 302.2, 402, 602.2, 640.1, 643.2, 648.2, 802.1, 802.2) assoziiert ist.

6. System (200, 300, 400, 600, 800) nach Anspruch 5, **dadurch gekennzeichnet, dass**
- das Peer-to-Peer-Modul (228.1, 328.1, 326.3, 428.1), das der ersten Entität (202, 302.1, 302.2, 402, 602.2, 640.1, 643.2, 648.2, 802.1, 802.2) zugeordnet ist, ein Verschlüsselungsmittel (436) umfasst, das so eingerichtet ist, dass der mindestens eine Telemetriedatensatz verschlüsselt wird,
- wobei das Peer-to-Peer-Modul (228.1, 328.1, 326.3, 428.1), das der ersten Entität (202, 302.1, 302.2, 402, 602.2, 640.1, 643.2, 648.2, 802.1, 802.2) zugeordnet ist, so eingerichtet ist, dass der verschlüsselte Telemetriedatensatz zusammen mit der Peer-to-Peer-Identifikation der ersten Entität (202, 302.1, 302.2, 402, 602.2, 640.1, 643.2, 648.2, 802.1, 802.2) an die Peer-to-Peer-Anwendung (224, 324, 424, 524) übertragen wird,
- wobei die Peer-to-Peer-Anwendung (224, 324, 424, 524) so eingerichtet ist, dass der empfangene verschlüsselte Telemetriedatensatz an das Auswertemittel (230, 330, 430.1, 430.2) auf der Grundlage der mit dem empfangenen Telemetriedatensatz assoziierten Peer-to-Peer-Identifikation weitergeleitet wird,
- wobei das Auswertemittel (230, 330, 430.1, 430.2) mindestens ein Entschlüsselungsmittel (438.1, 438.2) zum Entschlüsseln des weitergeleiteten Telemetriedatensatzes umfasst.

7. System (200, 300, 400, 600, 800) nach einem der vorherigen Ansprüche, **dadurch gekennzeichnet, dass**
- die mindestens eine erste Entität (202, 302.1, 302.2, 402, 602.2, 640.1, 643.2, 648.2, 802.1, 802.2) ein Fahrzeug ist (202, 302.1, 302.2, 402, 602.2, 640.1, 643.2, 648.2, 802.1, 802.2), umfassend mindestens ein Sensormodul (332), das so eingerichtet ist, dass mindestens ein auf das Fahrzeug bezogener Telemetrieparameter (202, 302.1, 302.2, 402, 602.2, 640.1, 643.2, 648.2, 802.1, 802.2) erfasst wird,
- wobei der erfasste Telemetrieparameter dem dem Fahrzeug (202, 302.1, 302.2, 402, 602.2, 640.1, 643.2, 648.2, 802.1, 802.2) zugeordneten Peer-to-Peer-Modul (228.1, 328.1, 326.3, 428.1) zur Verfügung gestellt wird.

8. System (200, 300, 400, 600, 800) nach einem der vorherigen Ansprüche, **dadurch gekennzeichnet, dass**
- die mindestens eine erste Entität (202, 302.1, 302.2, 402, 602.2, 640.1, 643.2, 648.2) ein medizintechnisches Gerät (202, 302.1, 302.2, 402, 602.2, 640.1, 643.2, 648.2, 802.1, 802.2) ist, umfassend mindestens ein Sensormodul (332), das so eingerichtet ist, dass mindestens ein Telemetrieparameter gemessen wird, der sich auf mindestens einen Benutzer des medizintechnischen Geräts (202, 302.1, 302.2, 402, 602.2, 640.1, 643.2, 648.2, 802.1, 802.2) bezieht,
- wobei der gemessene Telemetrieparameter dem dem medizintechnischen Gerät (202, 302.1, 302.2, 402, 402, 602.2, 640.1, 643.2, 648.2, 802.1, 802.2) zugeordneten Peer-to-Peer-Modul (228.1, 328.1, 326.3, 428.1) zur Verfügung gestellt wird.

9. System (200, 300, 400, 600, 800) nach einem der vorherigen Ansprüche, **dadurch gekennzeichnet, dass**
- die mindestens eine Peer-to-Peer-Anwendung (224, 324, 424, 524) ein dezentrales Register oder eine gemeinsam genutzte Datenbank ist,
- wobei die Peer-to-Peer-Anwendung (224, 324, 424, 524) so eingerichtet ist, dass Daten mit bestimmten Beweisen oder Signaturen gespeichert werden.

10. System (200, 300, 400, 600, 800) nach einem der vorherigen Ansprüche, **dadurch gekennzeichnet, dass** die mindestens eine Peer-to-Peer-Anwendung (224, 324, 424, 524) eine Blockchain oder ein dezentrales Ledger ist, das mindestens zwei miteinander gekoppelte Blöcke (551, 553, 555) umfasst.

11. System (200, 300, 400, 600, 800) nach einem der vorherigen Ansprüche, **dadurch gekennzeichnet, dass** mindestens ein Teil der Computerknoten (226.1, 226.2, 226.3, 326.1, 326.2, 326.3, 426.1, 426.2, 426.3) und/oder Teilnehmer (228.1, 228.2, 328.1, 328.2, 428.1, 428.2) des Peer-to-Peer-Netzwerks (222, 322, 422, 622, 822) so eingerichtet ist/sind, dass mindestens die Richtigkeit mindestens eines durchgeführten Auswerteprozesses validiert.

12. Verfahren zum Auswerten mindestens eines Telemetriedatensatzes, umfassend:
- Übertragen mindestens eines Telemetriedatensatzes, der sich auf mindestens eine erste Entität (202, 302.1, 302.2, 402, 602.2, 640.1, 643.2, 648.2, 802.1, 802.2) bezieht, von einem Peer-to-Peer-Modul (228.1, 328.1, 326.3, 428).1) der ersten Entität (202, 302.1, 302.2, 402, 602.2, 640.1, 643.2, 648.2, 802.1, 802.2) an mindestens eine Peer-to-Peer-Anwendung (224, 324, 424, 524) mindestens eines Peer-to-Peer-Netzwerks (222, 322, 422, 622, 822),
- zumindest Steuern eines Auswerteprozesses des Telemetriedatensatzes durch die Peer-to-Peer-Anwendung (224, 324, 424, 524), und
- Bereitstellen mindestens eines Auswerteergebnisses durch die Peer-to-Peer-Anwendung (224, 324, 424, 524) an mindestens eine zweite Entität (204, 304, 404, 604.2, 804), ohne den empfangenen Telemetriedatensatz bereitzustellen,
- wobei die Peer-to-Peer-Anwendung (224, 324, 424, 524) und mindestens ein Teil der Computerknoten (226.1, 226.2, 226.3, 326.1, 326.2, 426.1, 426.2) des Peer-to-Peer-Netzwerks (222, 322, 422, 622, 822) überwachen, ob unberechtigte Einheiten versuchen, auf die Telemetriedaten zuzugreifen, und
- wobei die Peer-to-Peer-Anwendung (224, 324, 424, 524) auf der Grundlage des Überwachungsergebnisses mindestens eine vordefinierte Aktion durchführen kann.

13. Peer-to-Peer-Anwendung (224, 324, 424, 524), umfassend:
- Mittel zum Empfangen mindestens eines Telemetriedatensatzes, der sich auf mindestens eine erste Entität (202, 302.1, 302.2, 402, 602.2, 640.1, 643.2, 648.2, 802.1, 802.2) bezieht,
- Mittel zum Steuern eines Auswertemittels (230, 330, 430.1, 430.2), das so eingerichtet ist, dass der mindestens eine Telemetriedatensatz gemäß mindestens einer vorgegebenen Auswerteregel ausgewertet wird, und
- Mittel zum Bereitstellen mindestens eines Auswerteergebnisses an mindestens eine zweite Entität (204, 304, 404, 604.2, 804), ohne den empfangenen Telemetriedatensatz bereitzustellen,
- wobei die Peer-to-Peer-Anwendung (224, 324, 424, 524) und mindestens ein Teil der Computerknoten (226.1, 226.2, 226.3, 326.1, 326.2, 426.1, 426.2) des Peer-to-Peer-Netzwerks (222, 322, 422, 622, 822) überwachen, ob unberechtigte Einheiten versuchen, auf die Telemetriedaten zuzugreifen, und
- wobei die Peer-to-Peer-Anwendung (224, 324, 424, 524) auf der Grundlage des Überwachungsergebnisses mindestens eine vordefinierte Aktion durchführen kann.

14. Peer-to-Peer-Modul (228.1, 328.1, 326.3, 428.1), das mindestens einer ersten Entität (202, 302.1, 302.2, 402, 602.2, 640.1, 643.2, 648.2, 802.1, 802.2) zugeordnet werden kann, umfassend:
- mindestens ein Eingabemittel eingerichtet zum Empfangen mindestens eines Telemetriedatensatzes, der sich auf die erste Entität (202, 302.1, 302.2, 402, 602.2, 640.1, 643.2, 648.2, 802.1, 802.2) bezieht,
- mindestens ein Verschlüsselungsmittel (438.1, 438.2) eingerichtet zum Verschlüsseln des empfangenen Telemetriedatensatzes, und
- mindestens ein Kommunikationsmittel eingerichtet zum Übertragen des verschlüsselten Telemetriedatensatzes zusammen mit einer eindeutigen Peer-to-Peer-Identifikation, die der ersten Entität (202, 302.1, 302.2, 402, 602.2, 640.1, 643.2, 648.2, 802.1, 802.2) zugeordnet ist zu mindestens einer Peer-to-Peer-Anwendung (224, 324, 424, 524) mindestens eines Peer-to-Peer-Netzwerks (222, 322, 422, 622, 822), so dass die Peer-to-Peer-Anwendung (224, 324, 424, 524) und mindestens ein Teil der Computerknoten (226.1, 226.2, 226.3, 326.1, 326.2, 426.1, 426.2) des Peer-to-Peer-Netzwerks (222, 322, 422, 622. 822) überwachen, ob unberechtigte Einheiten versuchen, auf die Telemetriedaten zuzugreifen, und
- wobei die Peer-to-Peer-Anwendung (224, 324, 424, 524) auf der Grundlage des Überwachungsergebnisses mindestens eine vordefinierte Aktion durchführen kann.

15. Medizintechnisches Gerät (802.1, 802.2), das mindestens ein Peer-to-Peer-Modul (228.1, 328.1, 326.3, 428.1) nach Anspruch 14 umfasst.

## Revendications

1. Système (200, 300, 400, 600, 800) pour évaluer des données de télémétrie, comprenant :
- au moins un module peer-to-peer (228.1, 328.1, 326.3, 428.1) attribué à au moins une première entité (202, 302.1, 302.2, 402, 602.2, 640.1, 643.2, 648.2, 802.1, 802.2),
- au moins un réseau peer-to-peer (222, 322, 422, 622, 822) avec au moins une application peer-to-peer (224, 324, 424, 524),
- dans lequel le module peer-to-peer (228.1, 328.1, 326.3, 428.1) est configuré pour transmettre au moins un jeu de données de télémétrie concernant la première entité (202, 302.1, 302.2, 402, 602.2, 640.1, 643.2, 648.2, 802.1, 802.2) à l'application peer-to-peer (224, 324, 424, 524),
- dans lequel l'application peer-to-peer (224, 324, 424, 524) est configurée au moins pour contrôler des moyens d'évaluation (230, 330, 430.1, 430.2) configurés pour évaluer l'au moins un jeu de données de télémétrie selon au moins une règle d'évaluation présélectionnée,
- dans lequel l'application peer-to-peer (224, 324, 424, 524) est configurée pour contrôler des moyens d'évaluation (230, 330, 430.1, 430.2) de sorte qu'au moins un résultat d'évaluation est fourni à l'au moins une deuxième entité (204, 304, 404, 604.2, 804) sans fournir le jeu de données de télémétrie reçu,
- dans lequel l'application peer-to-peer (224, 324, 424, 524) et au moins une partie de nœuds d'ordinateur (226.1, 226.2, 226.3, 326.1, 326.2, 426.1, 426.2) du réseau peer-to-peer (222, 322, 422, 622, 822) contrôlent si des unités non autorisées essaient d'accéder aux données de télémétrie, et
- dans lequel, sur base du résultat du contrôle, l'application peer-to-peer (224, 324, 424, 524) peut réaliser au moins une action prédéfinie.

2. Système (200, 300, 400, 600, 800) selon la revendication 1, **caractérisé en ce que**
- les moyens d'évaluation (230, 330, 430.1, 430.2) sont configurés en outre, pour générer au moins un message de protocole comprenant des données de protocole au sujet du processus d'évaluation effectué,
- dans lequel l'application peer-to-peer (224, 324, 424, 524) est configuré pour fournir le message de protocole à au moins l'une de la première entité (202, 302.1, 302.2, 402, 602.2, 640.1, 643.2, 648.2, 802.1, 802.2) et de la deuxième entité (204, 304, 404, 604.2, 804).

3. Système (200, 300, 400, 600, 800) selon la revendication 1 ou 2, **caractérisé en ce que** l'au moins un module peer-to-peer (228.1, 228.2, 328.1, 326.3, 383.2, 428.1, 428.2) affecté à l'au moins une de la première entité (202, 302.1, 302.2, 402, 602.2, 640.1, 643.2, 648.2, 802.1, 802.2) et de la deuxième entité (204, 304, 404, 604.2, 804) est configuré pour induire la génération d'au moins un accord de transaction d'évaluation au sujet au moins des termes d'entrée du jeu de données de télémétrie et/ou d'au moins une règle d'évaluation et/ou des termes de sortie du résultat d'évaluation.

4. Système (200, 300, 400, 600, 800) selon l'une quelconque des revendications précédentes, **caractérisé en ce que**
- l'application peer-to-peer (224, 324, 424, 524) comprend les moyens d'évaluation (230, 330, 430.1, 430.2), et/ou
- au moins une banque de données (334) pouvant être contrôlée par l'application peer-to-peer (224, 324, 424, 524) comprend les moyens d'évaluation (230, 330, 430.1, 430.2), et/ou
- au moins un nœud d'ordinateur (426.3) du réseau peer-to-peer (222, 322, 422, 622, 822) comprend les moyens d'évaluation (230, 330, 430.1, 430.2).

5. Système (200, 300, 400, 600, 800) selon l'une des revendications précédentes, **caractérisé en ce que**
- l'au moins un jeu de données de télémétrie concernant la première entité (202, 302.1, 302.2, 402, 602.2, 640.1, 643.2, 648.2, 802.1, 802.2) est associé à une identification peer-to-peer unique de la première entité (202, 302.1, 302.2, 402, 602.2, 640.1, 643.2, 648.2, 802.1, 802.2), et
- dans lequel l'au moins un résultat d'évaluation est associé à l'identification peer-to-peer unique de la première entité (202, 302.1, 302.2, 402, 602.2, 640.1, 643.2, 648.2, 802.1, 802.2).

6. Système (200, 300, 400, 600, 800) selon la revendication 5, **caractérisé en ce que**
- le module peer-to-peer (228.1, 328.1, 326.3, 428.1) affecté à la première entité (202, 302.1, 302.2, 402, 602.2, 640.1, 643.2, 648.2, 802.1, 802.2) comprend des moyens de cryptage (436) configurés pour crypter au moins l'une jeu de données de télémétrie,
- dans lequel le module peer-to-peer (228.1, 328.1, 326.3, 428.1) affecté à la première entité (202, 302.1, 302.2, 402, 602.2, 640.1, 643.2, 648.2, 802.1, 802.2) est configuré pour transmettre le jeu de donnée de télémétrie crypté avec l'identification peer-to-peer unique de la première entité (202, 302.1, 302.2, 402, 602.2, 640.1, 643.2, 648.2, 802.1, 802.2) à l'application peer-to-peer (224, 324, 424, 524),
- dans lequel l'application peer-to-peer (224, 324, 424, 524) est configurée pour transmettre le jeu de données de télémétrie crypté reçu au moyens d'évaluation (230, 330, 430.1, 430.2) sur base de l'identification peer-to-peer associée au jeu de données de télémétrie reçu,
- dans lequel les moyens d'évaluation (230, 330, 430.1, 430.2) comprennent au moins un moyen de décryptage (438.1, 438.2) pour décrypter le jeu de données de télémétrie transmis.

7. Système (200, 300, 400, 600, 800) selon l'une des revendications précédentes, **caractérisé en ce que**
- l'au moins une première entité (202, 302.1, 302.2, 402, 602.2, 640.1, 643.2, 648.2, 802.1, 802.2) est un véhicule (202, 302.1, 302.2, 402, 602.2, 640.1, 643.2, 648.2, 802.1, 802.2) comprenant au moins un module de capteur (332) configuré pour détecter au moins un paramètre de télémétrie concernant le véhicule (202, 302.1, 302.2, 402, 602.2, 640.1, 643.2, 648.2, 802.1, 802.2),
- dans lequel le paramètre de télémétrie détecté est fourni au module peer-to-peer (228.1, 328.1, 326.3, 428.1) affecté au véhicule (202, 302.1, 302.2, 402, 602.2, 640.1, 643.2, 648.2, 802.1, 802.2).

8. Système (200, 300, 400, 600, 800) selon l'une des revendications précédentes, **caractérisé en ce que**
- l'au moins une première entité (202, 302.1, 302.2, 402, 602.2, 640.1, 643.2, 648.2) est un dispositif de technologie médicale (202, 302.1, 302.2, 402, 602.2, 640.1, 643.2, 648.2, 802.1, 802.2) comprenant au moins un module capteur (332) configuré pour mesurer au moins un paramètre de télémétrie concernant au moins un utilisateur du dispositif de technologie médicale (202, 302.1, 302.2, 402, 602.2, 640.1, 643.2, 648.2, 802.1, 802.2),
- dans lequel le paramètre de télémétrie détecté est fourni au module peer-to-peer (228.1, 328.1, 326.3, 428.1) affecté au dispositif de technologie médicale (202, 302.1, 302.2, 402, 602.2, 640.1, 643.2, 648.2, 802.1, 802.2).

9. Système (200, 300, 400, 600, 800) selon l'une des revendications précédentes, **caractérisé en ce que**
- l'au moins une application peer-to-peer (224, 324, 424, 524) est un registre décentralisé ou une banque de données partagée,
- dans lequel l'application peer-to-peer (224, 324, 424, 524) est configurée pour enregistrer des données ayant certaines preuves ou signatures données.

10. Système (200, 300, 400, 600, 800) selon l'une des revendications précédentes, **caractérisé en ce que** l'au moins une application peer-to-peer (224, 324, 424, 524) est une chaine de blocs ou un ledger décentralisé comprenant au moins deux blocs (551, 553, 555) couplés l'un à l'autre.

11. Système (200, 300, 400, 600, 800) selon l'une des revendications précédentes, **caractérisé en ce qu'**au moins une partie des nœuds d'ordinateur (226.1, 226.2, 226.3, 326.1, 326.2, 326,3, 426.1, 426.2, 426.3) et/ou participants (228.1, 228.2, 328.1, 328.2, 428.1, 428.2) du réseau peer-to-peer (222, 322, 422, 622, 822) est/sont configuré(s) pour valider au moins l'exactitude d'au moins un processus d'évaluation réalisé.

12. Procédé d'évaluation d'au moins un jeu de données de télémétrie, comprenant :
- la transmission d'au moins un jeu de données de télémétrie concernant au moins un première entité (202, 302.1, 302.2, 402, 602.2, 640.1, 643.2, 648.2, 802.1, 802.2) depuis un module peer-to-peer (228.1, 328.1, 326.3, 428.1) affecté à la première entité (202, 302.1, 302.2, 402, 602.2, 640.1, 643.2, 648.2, 802.1, 802.2) vers au moins une application peer-to-peer (224, 324, 424, 524) d'au moins un réseau peer-to-peer (222, 322, 422, 622, 822),
- au moins le contrôle d'un procédé d'évaluation du jeu de données de télémétrie par l'application peer-to-peer (224, 324, 424, 524), et
- la fourniture d'au moins un résultat d'évaluation par l'application peer-to-peer (224, 324, 424, 524) à au moins une deuxième entité (204, 304, 404, 604.2, 804) sous fournir le jeu de données de télémétrie reçu,
- dans lequel l'application peer-to-peer (224, 324, 424, 524) et au moins une partie de nœuds d'ordinateur (226.1, 226.2, 226.3, 326.1, 326.2, 426.1, 426.2) du réseau peer-to-peer (222, 322, 422, 622, 822) contrôlent si des unités non autorisées essayent d'accéder aux données de télémétrie, et
- dans lequel, sur base du résultat du contrôle, l'application peer-to-peer (224, 324, 424, 524) peut effectuer au moins une action prédéfinie.

13. Application peer-to-peer (224, 324, 424, 524) comprenant :
- des moyens de reception d'au moins un jeu de données de télémétrie concernant au moins une première entité (202, 302.1, 302.2, 402, 602.2, 640.1, 643.2, 648.2, 802.1, 802.2),
- des moyens de contrôle des moyens d'évaluation (230, 330, 430.1, 430.2) configurés pour évaluer l'au moins un jeu de données de télémétrie selon au moins un règle d'évaluation pré-établie, et
- des moyens de fourniture d'au moins un résultat d'évaluation vers au moins une deuxième entité (204, 304, 404, 604.2, 804) sans fournir le le jeu de données de télémétrie reçu,
- dans lequel l'application peer-to-peer (224, 324, 424, 524) et au moins une partie de nœuds d'ordinateur (226.1, 226.2, 226.3, 326.1, 326.2, 426.1, 426.2) du réseau peer-to-peer (222, 322, 422, 622, 822) contrôlent si des unités non autorisées essayent d'accéder aux données de télémétrie, et
- dans lequel, sur base du résultat du contrôle, l'application peer-to-peer (224, 324, 424, 524) peut effectuer au moins une action prédéfinie.

14. Module peer-to-peer (228.1, 328.1, 326.3, 428.1) pouvant être affecté à au moins une première entité (202, 302.1, 302.2, 402, 602.2, 640.1, 643.2, 648.2, 802.1, 802.2), comprenant :
- au moins un moyen d'entrée configuré pour recevoir l'au moins un jeu de données de télémétrie concernant la première entité (202, 302.1, 302.2, 402, 602.2, 640.1, 643.2, 648.2, 802.1, 802.2),
- au moins un moyen de cryptage (438.1, 438.2) configuré pour crypter le jeu de données de télémétrie reçu, et
- au moins un moyen de communication configuré pour transmettre le jeu de données de télémétrie crypté avec une identification peer-to-peer unique affectée à la première entité (202, 302.1, 302.2, 402, 602.2, 640.1, 643.2, 648.2, 802.1, 802.2) vers au moins une application peer-to-peer (224, 324, 424, 524) de l'au moins un réseau peer-to-peer (222, 322, 422, 622, 822) de sorte que l'application peer-to-peer (224, 324, 424, 524) et au moins une partie des nœuds d'ordinateur (226.1, 226.2, 226.3, 326.1, 326.2, 426.1, 426.2) du réseau peer-to-peer (222, 322, 422, 622, 822) contrôlent si des unités non autorisées essayent d'accéder aux données de télémétrie, et
- dans lequel, sur base du résultat du contrôle, l'application peer-to-peer (224, 324, 424, 524) peut effectuer au moins une action prédéfinie.

15. Dispositif de technologie médicale (802.1, 802.2) comprenant au moins un module peer-to-peer (228.1, 328.1, 326.3, 428.1) selon la revendication 14.
